# EUROPEAN PATENT SPECIFICATION

(11) **EP 1 001 977 B1**
(45) Date of publication and mention of the grant of the patent: **05.10.2005**
(21) Application number: 99955223.5
(22) Date of filing: 03.06.1999
(51) Int. Cl.: C07K 7/08, A61K 51/08, G01N 33/68

(54) **RADIOLABELED PEPTIDES FOR THE DIAGNOSIS AND TREATMENT OF BREAST AND PROSTATE TUMORS AND METASTASES OF SUCH TUMORS**
RADIOAKTIVE MARKIERTE PEPTIDE FÜR DIE DIAGNOSE UND THERAPIE VON BRUST- UND PROSTATATUMOREN UND VON METASTASEN DIESER TUMORE
PEPTIDES RADIOMARQUES PERMETTANT DE DIAGNOSTIQUER ET DE TRAITER DES TUMEURS DU SEIN ET DE LA PROSTATE ET DES METASTASES DE CES TUMEURS

(30) Priority: 05.06.1998 US 88074 P; 08.06.1998 US 88517 P
(43) Date of publication of application: 24.05.2000
(73) Proprietor: MALLINCKRODT INC., St. Louis, MO 63134 (US)
(72) Inventor: REUBI, Jean-Claude, CH-3084 Wabern (CH); BREEMAN, Wout, A., NL-3271 XD Mijnsheerenland (NL); SRINIVASAN, Ananthachari, St. Charles, MO 63303 (US)
(74) Representative: Carpmaels & Ransford
(86) International application number: PCT/US1999/012414
(87) International publication number: WO 1999/062563

(56) References cited:
- WO-A-91/01144
- US-A- 5 750 646
- WAP BREMAN ET AL.: "[111In-DTPA0,Por1,Tyr4]bombesin: studies in vitro and in rats" JOURNAL OF NUCLEAR MEDICINE., vol. 39, no. 5 Supplement, 25 May 1998 (1998-05-25), page 62P XP002125543 SOCIETY OF NUCLEAR MEDICINE. NEW YORK. 1019691, US ISSN: 0161-5505 cited in the application
- TJ HOFFMAN ET AL.: "Rh-105 Bombesin Analogs: Selective in vivo Targeting of Prostate Cancer with a Therapeutic Radionuclide" JOURNAL OF NUCLEAR MEDICINE., vol. 39, no. 5 Supplement, 25 May 1998 (1998-05-25), page 222P XP002125544 SOCIETY OF NUCLEAR MEDICINE. NEW YORK. 1019691, US ISSN: 0161-5505 cited in the application
- NING LI ET AL.: "In-Vitro and in-vivo Characterization of a Th-105 Tetrathiamacrocyle Conjugate of a Bombesin Analogue" JOURNAL OF NUCLEAR MEDICINE., vol. 37, no. 5 Supplement, May 1996 (1996-05), page 61P XP002125545 SOCIETY OF NUCLEAR MEDICINE. NEW YORK. 1019691, US ISSN: 0161-5505 cited in the application
- TJ HOFFMAN ET AL.: "Uptake and Retention of a Rh-105 Labeled Bombesin Analogue in GRP Receptor Expressing Neoplasms: An In Vitro Study" JOURNAL OF NUCLEAR MEDICINE., vol. 38, no. 5 Supplement, May 1997 (1997-05), pages 188P-189P, XP002125546 SOCIETY OF NUCLEAR MEDICINE. NEW YORK. 1019691, US ISSN: 0161-5505 cited in the application

### SEQUENCE LISTING

<110> Breeman, W. A. P.
   Reubi, Jean-Claude
   Srinivasan, Ananthachar
   Mallinckrodt Inc.
<120> Radiolabeled Peptides for the Diagnosis and Treatment of Breast and Prostate Tumors and Metastases of Such Tumors
<130> 1670-225
<140> PCT/US 99/12414
   <141> 1999-06-03
<150> U.S. 60/088074
   <151> 1998-06-05
<150> U.S. 60/088517
   <151> 1998-06-08
<160> 85
<170> PatentIn Ver. 2.0
<210> 1
   <211> 14
   <212> PRT
   <213> Bombina orientalis
<220>
   <221> SITE
   <222> (1)
   <223> Xaa is pyroglutamyl (pGlu).
<220>
   <221> SITE
   <222> (14)
   <223> Xaa is Met ending with an amide (Met-NH2).
<400> 1
   Xaa Gln Arg Leu Gly Asn Gln Trp Ala Val Gly His Leu Xaa
   1               5                   10
<210> 2
   <211> 6
   <212> PRT
   <213> Artificial Sequence
<220>
   <223> Description of Artificial Sequence:Bombesin analog.
<400> 2
   Trp Ala Val Gly His Leu
   1               5
<210> 3
   <211> 14
   <212> PRT
   <213> Artificial Sequence
<220>
   <221> SITE
   <222> (1)
   <223> Xaa is pyroglutamyl (pGlu).
<220>
   <221> SITE
   <222> (14)
   <223> Xaa is Met ending with an amide (Met-NH2).
<220>
   <223> Description of Artificial Sequence:Bombesin analog.
<400> 3
   Xaa Gln Lys Leu Gly Asn Gln Trp Ala Val Gly His Leu Xaa
   1               5                   10
<210> 4
   <211> 8
   <212> PRT
   <213> Artificial Sequence
<220>
   <223> Description of Artificial Sequence:Bombesin analog.
<400> 4
   Gln Trp Ala Val Gly His Leu Met
    1               5
<210> 5
   <211> 15
   <212> PRT
   <213> Artificial Sequence
<220>
   <221> SITE
   <222> (1)
   <223> Xaa is L- or D-His.
<220>
   <221> SITE
   <222> (2)
   <223> Xaa (shown as AA1 in the specification) is a cyclic amino acid residue.
<220>
   <221> SITE
   <222> (4)
   <223> Xaa (shown as AA3 in the specification) is any L or D amino acid residue.
<220>
   <221> SITE
   <222> (5)
   <223> Xaa (shown as AA4 in the specification) is any L or D amino acid residue.
<220>
   <221> SITE
   <222> (15)
   <223> Xaa (shown as AA14 in the specification) is any D or L amino acid residue and ends with an amide (Xaa-NR2).
<220>
   <223> Description of Artificial Sequence:Bombesin analog.
<400> 5
   Xaa Xaa Gln Xaa Xaa Gly Asn Gln Trp Ala Val Gly His Leu Xaa
    1               5                   10                  15
<210> 6
   <211> 16
   <212> PRT
   <213> Artificial Sequence
<220>
   <221> SITE
   <222> (1)
   <223> Xaa is L- or D-His.
<220>
   <221> SITE
   <222> (2)
   <223> Xaa is beta-Asp.
<220>
   <221> SITE
   <222> (3)
   <223> Xaa (shown as AA1 in the specification) is a cyclic amino acid residue.
<220>
   <221> SITE
   <222> (5)
   <223> Xaa (shown as AA3 in the specification) is any D or L amino acid residue.
<220>
   <221> SITE
   <222> (6)
   <223> Xaa (shown as AA4 in the specification) is any D or L amino acid residue.
<220>
   <221> SITE
   <222> (16)
   <223> Xaa (shown as AA14 in the specification) is any D or L amino acid residue and ends with an amide (Xaa-NH2).
<220>
   <223> Description of Artificial Sequence:Bombesin analog.
<400> 6
   Xaa Xaa Xaa Gln Xaa Xaa Gly Asn Gln Trp Ala Val Gly His Leu Xaa
    1               5                  10                  15
<210> 7
   <211> 14
   <212> PRT
   <213> Artificial Sequence
<220>
   <221> SITE
   <222> (1)
   <223> Xaa is pyroglutamyl (pGlu).
<220>
   <221> SITE
   <222> (14)
   <223> Xaa is Met ending in an amide (Met-NH2).
<220>
   <223> Description of Artificial Sequence:Bombesin analog.
<400> 7
   Xaa Gln Arg Tyr Gly Asn Gln Trp Ala Val Gly His Leu Xaa
    1               5                   10
<210> 8
   <211> 14
   <212> PRT
   <213> Artificial Sequence
<220>
   <221> SITE
   <222> (1)
   <223> Xaa is pyroglutamyl (pGlu).
<220>
   <221> SITE
   <222> (14)
   <223> Xaa is Met ending in an amide (Met-NH2).
<220>
   <223> Description of Artificial Sequence:Bombesin analog.
<400> 8
   Xaa Gln Arg Tyr Gly Asn Gln Trp Ala Val Gly His Leu Xaa
    1               5                   10
<210> 9
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (14)
   <223> Xaa is Met ending with an amide (Met-NH2).
<220>
   <223> Description of Artificial Sequence:Bombesin analog.
<400> 9
   Xaa Gln Arg Tyr Gly Asn Gln Trp Ala Val Gly His Leu Xaa
    1               5                   10
<210> 10
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (14)
   <223> Xaa is Met ending in an amide (Met-NH2).
<220>
   <223> Description of Artificial Sequence:Bombesin analog.
<400> 10
   Xaa Gln Arg Tyr Gly Asn Gln Trp Ala Val Gly His Leu Xaa
   1 5 10
<210> 11
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (14)
   <223> Xaa is Met ending in an amide (Met-NH2).
<220>
   <223> Description of Artificial Sequence:Bombesin analog.
<400> 11
   Xaa Gln Arg Tyr Gly Asn Gln Trp Ala Val Gly His Leu Xaa
   1 5 10
<210> 12
   <211> 15
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (14)
   <223> Xaa is norleucine ending with an amide (Nle-NH2).
<220>
   <223> Description of Artificial Sequence:Bombesin analog.
<400> 12
   Xaa Pro Gln Arg Tyr Gly Asn Gln Trp Ala Val Gly His Leu Xaa
    1               5                  10                  15
<210> 13
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (14)
   <223> Xaa is norleucine ending in an amide (Nle-NH2).
<220>
   <223> Description of Artificial Sequence:Bombesin analog.
<400> 13
   Xaa Gln Arg Tyr Gly Asn Gln Trp Ala Val Gly His Leu Xaa
    1               5                  10
<210> 14
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (14)
   <223> Xaa is norleucine ending with an amide (Nle-NH2).
<220>
   <223> Description of Artificial Sequence:Bombesin analog.
<400> 14
   Xaa Gln Arg Tyr Gly Asn Gln Trp Ala Val Gly His Leu Xaa
    1               5                  10
<210> 15
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <223> Description of Artificial Sequence:Bombesin analog.
<220>
   <221> SITE
   <222> (14)
   <223> Xaa is Met ending in an amide (Met-NH2).
<400> 15
   Xaa Gln Arg Tyr Gly Asn Gln Trp Ala Val Gly His Leu Xaa
    1               5                   10
<210> 16
   <211> 14
   <212> PRT
   <213> Artificial Sequence
<220>
   <221> SITE
   <222> (1)
   <223> Xaa is Mtc (thiomorpholino 2-carboxylic acid) with DTPA (diethylene triamine penta-acetic acid).
<220>
   <221> SITE'
   <222> (14)
   <223> Xaa is Met ending in an amide (Met-NH2).
<220>
   <223> Description of Artificial Sequence:Bombesin analog.
<400> 16
   Xaa Gln Arg Tyr Gly Asn Gln Trp Ala Val Gly His Leu Xaa
    1               5                   10
<210> 17
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinophenylalanine.
<220>
   <221> SITE
   <222> (14)
   <223> Xaa is Met ending with an amide (Met-NH2).
<220>
   <223> Description of Artificial Sequence:Bombesin analog.
<400> 17
   Xaa Gln Xaa Tyr Gly Asn Gln Trp Ala Val Gly His Leu Xaa
    1               5                  10
<210> 18
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinomethylphenylalanine.
<220>
   <221> SITE
   <222> (14)
   <223> Xaa is Met ending with an amide (Met-NH2).
<220>
   <223> Description of Artificial Sequence:Bombesin analog.
<400> 18
   Xaa Gln Xaa Tyr Gly Asn Gln Trp Ala Val Gly His Leu Xaa
    1               5                  10
<210> 19
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinoethylphenylalanine.
<220>
   <221> SITE
   <222> (14)
   <223> Xaa is Met ending with an amide (Met-NH2).
<220>
   <223> Description of Artificial Sequence:Bombesin analog.
<400> 19
   Xaa Gln Xaa Tyr Gly Asn Gln Trp Ala Val Gly His Leu Xaa
    1               5                   10
<210> 20
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinophenylalanine.
<220>
   <221> SITE
   <222> (14)
   <223> Xaa is Met ending with an amide (Met-NH2).
<220>
   <223> Description of Artificial Sequence:Bombesin analog.
<400> 20
   Xaa Gln Xaa Tyr Gly Asn Gln Trp Ala Val Gly His Leu Xaa
    1               5                   10
<210> 21
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinomethylphenylalanine.
<220>
   <221> SITE
   <222> (14)
   <223> Xaa is Met ending with an amide (Met-NH2).
<220>
   <223> Description of Artificial Sequence:Bombesin analog.
<400> 21
   Xaa Gln Xaa Tyr Gly Asn Gln Trp Ala Val Gly His Leu Xaa
    1               5                   10
<210> 22
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinoethylphenylalanine.
<220>
   <221> SITE
   <222> (14)
   <223> Xaa is Met ending with an amide (Met-NH2).
<220>
   <223> Description of Artificial Sequence:Bombesin analog.
<400> 22
   Xaa Gln Xaa Tyr Gly Asn Gln Trp Ala Val Gly His Leu Xaa
    1               5                   10
<210> 23
   <211> 14
   <212> PRT
   <213> Artificial Sequence
<220>
   <221> SITE
   <222> (1)
   <223> Xaa is hPip (hombpipecolic acid) with DTPA (diethylene triamine penta-acetic acid).
<220>
   <221> SITE
   <222> (3)
   <223> Xaa is guanidinophenylalanine.
<220>
   <221> SITE
   <222> (14)
   <223> Xaa is Met ending with an amide (Met-NH2).
<220>
   <223> Description of Artificial Sequence:Bombesin analog.
<400> 23
   Xaa Gln Xaa Tyr Gly Asn Gln Trp Ala Val Gly His Leu Xaa
    1               5                  10
<210> 24
   <211> 14
   <212> PRT
   <213> Artificial Sequence.
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinomethylphenylalanine.
<220>
   <221> SITE
   <222> (14)
   <223> Xaa is Met ending with an amide (Met-NH2).
<220>
   <223> Description of Artificial Sequence:Bombesin analog.
<400> 24
   Xaa Gln Xaa Tyr Gly Asn Gln Trp Ala Val Gly His Leu Xaa
    1               5                  10
<210> 25
   <211> 14
   <212> PRT
   <213> Artificial Sequence
<220>
   <221> SITE
   <222> (1)
   <223> Xaa is Mtc (thiomorpholino 2-carboxylic acid) with DTPA (diethylene triamine penta-acetic acid).
<220>
   <221> SITE
   <222> (3)
   <223> Xaa is guanidinoethylphenylalanine.
<220>
   <221> SITE
   <222> (14)
   <223> Xaa is Met ending with an amide (Met-NH2).
<220>
   <223> Description of Artificial Sequence:Bombesin analog.
<400> 25
   Xaa Gln Xaa Tyr Gly Asn Gln Trp Ala Val Gly His Leu Xaa
    1               5                  10
<210> 26
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinocyclopentylalanine.
<220>
   <221> SITE
   <222> (14)
   <223> Xaa is Met ending with an amide (Met-NH2).
<220>
   <223> Description of Artificial Sequence:Bombesin analog.
<400> 26
   Xaa Gln Xaa Tyr Gly Asn Gln Trp Ala Val Gly His Leu Xaa
    1               5                  10
<210> 27
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinomethylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 27
   Xaa Gln Xaa Tyr Gly Asn Gln Trp Ala Val Gly His Leu Xaa
    1               5                  10
<210> 28
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinoethylcyclopentylalanine.
<220>
   <221> SITE
   <222> (14)
   <223> Xaa is Met ending with an amide (Met-NH2).
<220>
   <223> Description of Artificial Sequence:Hombesin analog.
<400> 28
   Xaa Gln Xaa Tyr Gly Asn Gln Trp Ala Val Gly His Leu Xaa
    1               5                  10
<210> 29
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinopropylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 29
   Xaa Gln Xaa Tyr Gly Asn Gln Trp Ala Val Gly His Leu Xaa
    1               5                  10
<210> 30
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinocyclohexylalanine.
<220>
   <221> SITE
   <222> (14)
   <223> Xaa is Met ending with an amide (Met-NH2).
<220>
   <223> Description of Artificial Sequence:Bombesin analog.
<400> 30
   Xaa Gln Xaa Tyr Gly Asn Gln Trp Ala Val Gly His Leu Xaa
    1               5                  10
<210> 31
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinomethylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 31
   Xaa Gln Xaa Tyr Gly Asn Gln Trp Ala Val Gly His Leu Xaa
    1               5                  10
<210> 32
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinoethylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 32
   Xaa Gln Xaa Tyr Gly Asn Gln Trp Ala Val Gly His Leu Xaa
    1               5                  10
<210> 33
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinopropylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 33
   Xaa Gln Xaa Tyr Gly Asn Gln Trp Ala Val Gly His Leu Xaa
    1               5                  10
<210> 34
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinocycloheptylalanine.
<220>
   <221> SITE
   <222> (14)
   <223> Xaa is Met ending with an amide (Met-NH2).
<220>
   <223> Description of Artificial Sequence:Bombesin analog.
<400> 34
   Xaa Gln Xaa Tyr Gly Asn Gln Trp Ala Val Gly His Leu Xaa
    1               5                  10
<210> 35
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinomethylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 35
   Xaa Gln Xaa Tyr Gly Asn Gln Trp Ala Val Gly His Leu Xaa
    1               5                  10
<210> 36
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinoethylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 36
   Xaa Gln Xaa Tyr Gly Asn Gln Trp Ala Val Gly His Leu Xaa
    1               5                  10
<210> 37
   <211> 14
   <212> PRT
   <213> Artificial Sequence
<220>
   <221> SITE
   <222> (1)
   <223> Xaa is Pro with DTPA (diethylene triamine penta-acetic acid).
<220>
   <221> SITE
   <222> (3)
   <223> Xaa is guanidinopropylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 37
   Xaa Gln Xaa Tyr Gly Asn Gln Trp Ala Val Gly His Leu Xaa
    1               5                   10
<210> 38
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinocyclopentylalaninine.
<220>
   <221> SITE
   <222> (14)
   <223> Xaa is Met ending with an amide (Met-NH2).
<220>
   <223> Description of Artificial Sequence:Bombesin analog.
<400> 38
   Xaa Gln Xaa Tyr Gly Asn Gln Trp Ala Val Gly His Leu Xaa
    1               5                   10
<210> 39
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinomethylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 39
   Xaa Gln Xaa Tyr Gly Asn Gln Trp Ala Val Gly His Leu Xaa
    1               5                  10
<210> 40
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinoethylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 40
   Xaa Gln Xaa Tyr Gly Asn Gln Trp Ala Val Gly His Leu Xaa
    1               5                  10
<210> 41
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinopropylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 41
   Xaa Gln Xaa Tyr Gly Asn Gln Trp Ala Val Gly His Leu Xaa
    1               5                  10
<210> 42
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinocyclohexylalanine.
<220>
   <221> SITE
   <222> (14)
   <223> Xaa is Met ending with an amide (Met-NH2).
<220>
   <223> Description of Artificial Sequence:Bombesin analog.
<400> 42
   Xaa Gln Xaa Tyr Gly Asn Gln Trp Ala Val Gly His Leu Xaa
    1               5                  10
<210> 43
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinomethylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 43
   Xaa Gln Xaa Tyr Gly Asn Gln Trp Ala Val Gly His Leu Xaa
    1               5                  10
<210> 44
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinoethylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 44
   Xaa Gln Xaa Tyr Gly Asn Gln Trp Ala Val Gly His Leu Xaa
    1               5                  10
<210> 45
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinopropylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 45
   Xaa Gln Xaa Tyr Gly Asn Gln Trp Ala Val Gly His Leu Xaa
    1               5                  10
<210> 46
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinocycloheptylalanine.
<220>
   <221> SITE
   <222> (14)
   <223> Xaa is Met ending with an amide (Met-NH2).
<220>
   <223> Description of Artificial Sequence:Bombesin analog.
<400> 46
   Xaa Gln Xaa Tyr Gly Asn Gln Trp Ala Val Gly His Leu Xaa
    1               5                  10
<210> 47
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinomethylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 47
   Xaa Gln Xaa Tyr Gly Asn Gln Trp Ala Val Gly His Leu Xaa
    1               5                  10
<210> 48
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinoethylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 48
   Xaa Gln Xaa Tyr Gly Asn Gln Trp Ala Val Gly His Leu Xaa
    1               5                  10
<210> 49
   <211> 14
   <212> PRT
   <213> Artificial Sequence
<220>
   <221> SITE
   <222> (1)
   <223> Xaa is Pip (pipecolic acid) with DTPA (diethylene triamine penta-acetic acid).
<220>
   <221> SITE
   <222> (3)
   <223> Xaa is guanidinopropylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 49
   Xaa Gln Xaa Tyr Gly Asn Gln Trp Ala Val Gly His Leu Xaa
    1               5                   10
<210> 50
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (3)
   <223> Xaa is guanidinocyclopentylalanine.
<220>
   <221> SITE
   <222> (14)
   <223> Xaa is Met ending with an amide (Met-NH2).
<220>
   <223> Description of Artificial Sequence:Bombesin analog.
<400> 50
   Xaa Gln Xaa Tyr Gly Asn Gln Trp Ala Val Gly His Leu Xaa
    1               5                   10
<210> 51
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (3)
   <223> Xaa is guanidinomethylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 51
   Xaa Gln Xaa Tyr Gly Asn Gln Trp Ala Val Gly His Leu Xaa
    1               5                  10
<210> 52
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (3)
   <223> Xaa is guanidinoethylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 52
   Xaa Gln Xaa Tyr Gly Asn Gln Trp Ala Val Gly His Leu Xaa
    1               5                  10
<210> 53
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (3)
   <223> Xaa is guanidinopropylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 53
   Xaa Gln Xaa Tyr Gly Asn Gln Trp Ala Val Gly His Leu Xaa
    1               5                  10
<210> 54
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (3)
   <223> Xaa is guanidinocyclohexylalanine.
<220>
   <221> SITE
   <222> (14)
   <223> Xaa is Met ending with an amide (Met-NH2).
<220>
   <223> Description of Artificial Sequence:Bombesin analog.
<400> 54
   Xaa Gln Xaa Tyr Gly Asn Gln Trp Ala Val Gly His Leu Xaa
    1               5                  10
<210> 55
   <211> 14
   <212> PRT
   <213> Artificial Sequence
<220>
   <221> SITE
   <222> (1) .
   <223> Xaa is hPip (homopipecolic acid) with DTPA (diethylene triamine penta-acetic acid).
<220>
   <221> SITE
   <222> (3)
   <223> Xaa is guanidinomethylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 55
   Xaa Gln Xaa Tyr Gly Asn Gln Trp Ala Val Gly His Leu Xaa
    1               5                  10
<210> 56
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (3)
   <223> Xaa is guanidinoethylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 56
   Xaa Gln Xaa Tyr Gly Asn Gln Trp Ala Val Gly His Leu Xaa
    1               5                  10
<210> 57
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (3)
   <223> Xaa is guanidinopropylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 57
   Xaa Gln Xaa Tyr Gly Asn Gln Trp Ala Val Gly His Leu Xaa
    1               5                  10
<210> 58
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (3)
   <223> Xaa is guanidinocycloheptylalanine.
<220>
   <221> SITE
   <222> (14)
   <223> Xaa is Met ending with an amide (Met-NH2).
<220>
   <223> Description of Artificial Sequence:Bombesin analog.
<400> 58
   Xaa Gln Xaa Tyr Gly Asn Gln Trp Ala Val Gly His Leu Xaa
    1               5                  10
<210> 59
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (3)
   <223> Xaa is guanidinomethylcycloheptylalahine.
<220>
   <221> SITE
   <222> (14)
   <223> Xaa is Met ending with an amide (Met-NH2).
<220>
   <223> Description of Artificial Sequence:Bombesin analog.
<400> 59
   Xaa Gln Xaa Tyr Gly Asn Gln Trp Ala Val Gly His Leu Xaa
    1               5                  10
<210> 60
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (3)
   <223> Xaa is guanidinoethylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 60
   Xaa Gln Xaa Tyr Gly Asn Gln Trp Ala Val Gly His Leu Xaa
    1               5                  10
<210> 61
   <211> 14
   <212> PRT
   <213> Artificial Sequence
<220>
   <221> SITE
   <222> (1)
   <223> Xaa is hPip (homopipecolic acid) with DTPA (diethylene triamine penta-acetic acid).
<220>
   <221> SITE
   <222> (3)
   <223> Xaa is guanidinopropylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 61
   Xaa Gln Xaa Tyr Gly Asn Gln Trp Ala Val Gly His Leu Xaa
    1               5                  10
<210> 62
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinocyclopentylalanine.
<220>
   <221> SITE
   <222> (14)
   <223> Xaa is Met ending with an amide (Met-NH2).
<220>
   <223> Description of Artificial Sequence:Bombesin analog.
<400> 62
   Xaa Gln Xaa Tyr Gly Asn Gln Trp Ala Val Gly His Leu Xaa
    1               5                  10
<210> 63
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinomethylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 63
   Xaa Gln Xaa Tyr Gly Asn Gln Trp Ala Val Gly His Leu Xaa
    1               5                   10
<210> 64
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinoethylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 64
   Xaa Gln Xaa Tyr Gly Asn Gln Trp Ala Val Gly His Leu Xaa
    1               5                   10
<210> 65
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinopropylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 65
   Xaa Gln Xaa Tyr Gly Asn Gln Trp Ala Val Gly His Leu Xaa
    1               5                   10
<210> 66
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinocyclohexylalanine.
<220>
   <221> SITE
   <222> (14)
   <223> Xaa is Met ending with an amide (Met-NH2).
<220>
   <223> Description of Artificial Sequence:Bombesin analog.
<400> 66
   Xaa Gln Xaa Tyr Gly Asn Gln Trp Ala Val Gly His Leu Xaa
    1               5                   10
<210> 67
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinomethylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 67
   Xaa Gln Xaa Tyr Gly Asn Gln Trp Ala Val Gly His Leu Xaa
    1               5                   10
<210> 68
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinoethylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 68
   Xaa Gln Xaa Tyr Gly Asn Gln Trp Ala Val Gly His Leu Xaa
    1               5                   10
<210> 69
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinopropylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 69
   Xaa Gln Xaa Tyr Gly Asn Gln Trp Ala Val Gly His Leu Xaa
    1               5                  10
<210> 70
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinocycloheptylalanine.
<220>
   <221> SITE
   <222> (14)
   <223> Xaa is Met ending with an amide (Met-NH2).
<220>
   <223> Description of Artificial Sequence:Bombesin analog.
<400> 70
   Xaa Gln Xaa Tyr Gly Asn Gln Trp Ala Val Gly His Leu Xaa
    1               5                  10
<210> 71
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinomethylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 71
   Xaa Gln Xaa Tyr Gly Asn Gln Trp Ala Val Gly His Leu Xaa
    1               5                  10
<210> 72
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinoethylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 72
   Xaa Gln Xaa Tyr Gly Asn Gln Trp Ala Val Gly His Leu Xaa
    1               5                  10
<210> 73
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinopropylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 73
   Xaa Gln Xaa Tyr Gly Asn Gln Trp Ala Val Gly His Leu Xaa
    1               5                  10
<210> 74
   <211> 14
   <212> PRT
   <213> Artificial Sequence
<220>
   <221> SITE
   <222> (1)
   <223> Xaa is Mtc (thiomorpholino 2-carboxylic acid) with DTPA (diethylene triamine penta-acetic acid).
<220>
   <221> SITE
   <222> (3)
   <223> Xaa is guanidinocyclopentylalanine.
<220>
   <221> SITE
   <222> (14)
   <223> Xaa is Met ending with an amide (Met-NH2).
<220>
   <223> Description of Artificial Sequence:Bombesin analog.
<400> 74
   Xaa Gln Xaa Tyr Gly Asn Gln Trp Ala Val Gly His Leu Xaa
    1               5                  10
<210> 75
   <211> 14
   <212> PRT
   <213> Artificial Sequence
<220>
   <221> SITE
   <222> (1)
   <223> Xaa is Mtc (thiomorpholino 2-carboxylic acid) with DTPA (diethylene triamine penta-acetic acid).
<220>
   <221> SITE
   <222> (3)
   <223> Xaa is guanidinomethylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 75
   Xaa Gln Xaa Tyr Gly Asn Gln Trp Ala Val Gly His Leu Xaa
    1               5                   10
<210> 76
   <211> 14
   <212> PRT
   <213> Artificial Sequence
<220>
   <221> SITE
   <222> (1)
   <223> Xaa is Mtc (thiomorpholino 2-carboxylic acid) with DTPA (diethylene triamine penta-acetic acid).
<220>
   <221> SITE
   <222> (3)
   <223> Xaa is guanidinoethylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 76
   Xaa Gln Xaa Tyr Gly Asn Gln Trp Ala Val Gly His Leu Xaa
    1               5                   10
<210> 77
   <211> 14
   <212> PRT
   <213> Artificial Sequence
<220>
   <221> SITE
   <222> (1)
   <223> Xaa is Mtc (thiomorpholino 2-carboxylic acid) with DTPA (diethylene triamine penta-acetic acid).
<220>
   <221> SITE
   <222> (3)
   <223> Xaa is guanidinopropylcyclopentylalanine.
<220>
   <221> SITE
   <222> (14)
   <223> Xaa is Met ending with an amide (Met-NH2).
<220>
   <223> Description of Artificial Sequence:Bombesin analog.
<400> 77
   Xaa Gln Xaa Tyr Gly Asn Gln Trp Ala Val Gly His Leu Xaa
    1               5                   10
<210> 78
   <211> 14
   <212> PRT
   <213> Artificial Sequence
<220>
   <221> SITE
   <222> (1)
   <223> Xaa is Mtc (thiomorpholino 2-carboxylic acid) with DTPA (diethylene triamine penta-acetic acid).
<220>
   <221> SITE
   <222> (3)
   <223> Xaa is guanidinocyclohexylalanine.
<220>
   <221> SITE
   <222> (14)
   <223> Xaa is Met ending with an amide (Met-NH2).
<220>
   <223> Description of Artificial Sequence:Bombesin analog.
<400> 78
   Xaa Gln Xaa Tyr Gly Asn Gln Trp Ala Val Gly His Leu Xaa
    1               5                   10
<210> 79
   <211> 14
   <212> PRT
   <213> Artificial Sequence
<220>
   <221> SITE
   <222> (1)
   <223> Xaa is Mtc (thiomorpholino 2-carboxylic acid) with DTPA (diethylene triamine penta-acetic acid).
<220>
   <221> SITE
   <222> (3)
   <223> Xaa is guanidinomethylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 79
   Xaa Gln Xaa Tyr Gly Asn Gln Trp Ala Val Gly His Leu Xaa
    1               5                  10
<210> 80
   <211> 14
   <212> PRT
   <213> Artificial Sequence
<220>
   <221> SITE
   <222> (1)
   <223> Xaa is Mtc (thiomorpholino 2-carboxylic acid) with DTPA (diethylene triamine penta-acetic acid).
<220>
   <221> SITE
   <222> (3)
   <223> Xaa is guanidinoethylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 80
   Xaa Gln Xaa Tyr Gly Asn Gln Trp Ala Val Gly His Leu Xaa
    1               5                  10
<210> 81
   <211> 14
   <212> PRT
   <213> Artificial Sequence
<220>
   <221> SITE
   <222> (1)
   <223> Xaa is Mtc (thiomorpholino 2-carboxylic acid) with DTPA (diethylene triamine penta-acetic acid).
<220>
   <221> SITE
   <222> (3)
   <223> Xaa is guanidinopropylcyclohexylalanine.
<220>
   <221> SITE
   <222> (14)
   <223> Xaa is Met ending with an amide (Met-NH2).
<220>
   <223> Description of Artificial Sequence:Bombesin analog.
<400> 81
   Xaa Gln Xaa Tyr Gly Asn Gln Trp Ala Val Gly His Leu Xaa
    1               5                   10
<210> 82
   <211> 14
   <212> PRT
   <213> Artificial Sequence
<220>
   <221> SITE
   <222> (1)
   <223> Xaa is Mtc (thiomorpholino 2-carboxylic acid) with DTPA (diethylene triamine penta-acetic acid).
<220>
   <221> SITE
   <222> (3)
   <223> Xaa is guanidinocycloheptylalanine.
<220>
   <221> SITE
   <222> (14)
   <223> Xaa is Met ending with an amide (Met-NH2).
<220>
   <223> Description of Artificial Sequence:Bombesin analog.
<400> 82
   Xaa Gln Xaa Tyr Gly Asn Gln Trp Ala Val Gly His Leu Xaa
    1               5                   10
<210> 83
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinomethylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 83
   Xaa Gln Xaa Tyr Gly Asn Gln Trp Ala Val Gly His Leu Xaa
    1               5                   10
<210> 84
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinoethylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 84
   Xaa Gln Xaa Tyr Gly Asn Gln Trp Ala Val Gly His Leu Xaa
    1               5                   10
<210> 85
   <211> 14
   <212> PRT
   <213> Artificial Sequence
<220>
   <221> SITE
   <222> (1)
   <223> Xaa is Moc (morpholino 2-carboxylic acid) with DTPA (diethylene triamine penta-acetic acid).
<220>
   <221> SITE
   <222> (3)
   <223> Xaa is guanidinopropylcycloheptylalanine.
<220>
   <221> SITE
   <222> (14)
   <223> Xaa is Met ending with an amide (Met-NH2).
<220>
   <223> Description of Artificial Sequence:Bombesin analog.
<400> 85
   Xaa Gln Xaa Tyr Gly Asn Gln Trp Ala Val Gly His Leu Xaa
    1               5                  10

## Description

### BACKGROUND OF THE INVENTION

This invention relates to compounds and methods for the diagnosis and treatment of tumors of the breast and prostate, as well as metastases of such tumors.

Many tumors have biochemical receptors that cause certain molecules, typically peptides or proteins, to bind to the tumor. One approach to diagnose tumors is to identify a compound that binds to a particular tumor, radiolabel the compound with a suitable radionuclide, administer the compound to the patient (generally via intravenous injection), allow the compound to become bound to the tumor, and then image the location where the radioactive decay occurs. While the concept as thus presented is rather simple, in practice it is quite difficult. The first challenge is to identify a candidate compound. If the compound does not bind very strongly to the tumor and for a sufficient period of time, it will not be possible to obtain adequate diagnosis. Moreover, even if the compound binds to the tumor, if it also binds to surrounding healthy tissue, the diagnosis will be difficult or impossible. Further, it is necessary that the linkage of the compound to the radionuclide not disturb the affinity of the tumor for the compound. Another issue is the potential toxicity of the compound in the patient. Some compounds that may be very suitable from a binding perspective, may be too toxic to use.

A similar approach can be taken in tumor therapy, where one would identify a suitable compound, radiolabel the compound with a suitable radionuclide, and administer the compound to the patient (generally via intravenous injection, but possibly by direct injection into the tumor mass). The radionuclide will then decay, releasing energy to kill or reduce the growth of the tumor. Again, the concept is simple, but in practice there are many difficulties. In addition to the problems mentioned above, it is necessary that there be very little of the compound anywhere in the body except in the tumor, because of the danger of the high energy radiation to healthy tissue. This means that not only must the compound bind very strongly to the tumor, but there must be no or very little binding to healthy tissues, even if they are not in the vicinity of the tumor.

Attempts to locate suitable compounds have been fraught with difficulty. Because it is desirable to screen large numbers of potential compounds quickly, various "shortcut" assays and models have been developed. Unfortunately, many of these techniques have produced incorrect or misleading data.

Many researchers have used cell line cultures to screen compounds. While the use of cell lines as a screening technique has advantages, it has been found that cell line cultures often have binding affinity for compounds that is not exhibited by actual tumors. Thus, data from this technique produces false positives.

Other researchers have used homogenates of tumors, where a sample of the tumor has been subjected to high shear in a laboratory blender. One problem with this technique is that not only the tumor, but any surrounding tissues that were attached to the tumor are included in the homogenate, thus rendering it impossible to know if any binding affinity is from the tumor or from the surrounding tissues. Further, the shear of the homogenization breaks open the cell membranes, allowing for the possibility of binding that would not occur in an intact cell.

A screening method that produces unambiguous results is a morphological study in which sections (thin slices) of a tumor and surrounding tissue are contacted with a candidate compound that has been labeled with a radionuclide that is suitable for exposing photographic film. This technique clearly differentiates between receptors that are present in the tumor and those present in the surrounding tissue. Unfortunately, this technique is very labor intensive and depends on having suitable tumor tissue samples available.

Peptides and other compounds have been used without radiolabeling to affect the growth of tumors. While some such compounds may also be useful with radiolabeling for imaging and radiotherapy, the correlation between compounds useful for chemotherapy and those useful for radiotherapy is very low.

Bombesin is a peptide originally isolated from frog skin. It is an example of a compound that binds to GRP (Gastrin Releasing Peptide) receptors. Bombesin has the structure:

pGlu-Gln-Arg-Leu-Gly-Asn-Gln-Trp-Ala-Val-Gly-His-Leu-Met-NH₂

Considerable work has been conducted in an attempt to identify tumor and non-tumor GRP receptors. Unfortunately, much of that work has yielded results that are inaccurate or misleading.

Breeman et al. "[¹¹¹In-DTPA⁰, Pro¹, Tyr⁴]bombesin: Studies *In Vitro* and in Rats", J. Nucl. Med. 39 (1998) 62P teach that high and specific uptake was found in the pancreas and tissues of the GI-tract. Uptake was blocked by iv co-injection of 100 ug of Tyr⁴-BN with the radiolabeled peptide, but not when administered 1 hour after the radiotracer indicating the internalization of the radioligand.

Hoffman, Li. Sieckman, and Volkert, "Uptake and Retention of a Rh-105 Bombesin Analogue in GRP Receptor Expressing Neoplasms: An *In Vitro* Study", *JNM* 38 (1997) 188P-189P (abstract) teach that the affinity of a Rh-105 labeled bombesin analog for the GRP receptor was investigated along with its prolonged cellular retention in the PC-3 human prostate cancer cell line (65% @ 2 h) and CF-PAC1 human pancreatic cell line (41% @ 2 h). The Rh-105 analog was rapidly internalized intracellularly in both cell lines studied. The author states that the selective affinity & prolonged retention in neoplastic cells make this radiolabeled peptide a potential candidate for radiotherapy.

Hoffman, Li, ,Volkert, Sieckman, Higginbotham, and Ochrymowycz, "Synthesis and Characterization of Rh-105 Labelled Bombesin Analogues: Enhancement of GRP Binding Affinity Utilizing Aliphatic Carbon Chain Linkers", *Journal of Labelled Compounds and Radiopharmaceuticals* 40 (1997) 490-492 (abstract) teach that the IC₅₀ values (using Swiss 3T3 fibroblasts) were determined for a series of 4 peptides and the non-metalated sulfur macrocyclic analogs expressed similar affinities to the GRP receptor than the parent BBN peptide. Upon Rh(III) complexation, decreasing the proximity of the Rh(III)Cl₂-16 and S₄ complex to the binding region of BBN, increases the affinity of the final metalated peptide for the GRP receptor. This data may have implications in preparing other metalated BBN analogues which maintain specificity and high affinity for GRP receptors expressed on neoplastic cells.

Hoffman, Sieckman, Ochrymowycz, Higginbotham, Volkert, and Ketring, "*In Vitro* and *In Vivo* Characterization of a Rh-105-Tetrathiamacrocycle Conjugate of a Labelled Bombesin Analogue", J. Nucl. Med. 37 (1996) 61 P teach that biodistribution studies of the Rh-105 analogue in normal mice showed predominant clearance into the urine and low retention in the kidneys. Data demonstrate the feasibility of forming Rh-105 conjugates with BBN analogs as potential therapeutic agents that specifically target neoplastic cells expressing BBN2 receptors.

Hoffman et al. "Rh-105 Bombesin Analogs: Selective *In Vivo* Targeting of Prostate Cancer with a Radionuclide". J. Nucl. Med. 39 (1998) 982P teach that an Rh-105 BBN (7-14) analog, with a 4 carbon spacer between the sulfur macrocycle and the Q amino acid was evaluated in nude mice possessing PC-3 prostatic tumors. Tumor/Muscle ratios were 7.8. 7.7. and 13.6 @ 4, 24, & 48 hours p.i., respectively. The conclusion is that the selective affinity and prolonged retention of this radiolabeled peptide in prostatic cancer cells makes it an attractive candidate for radiotherapy.

T.J. Hoffman, G.L Sieckman, and W.A. Volkert, " Iodinated Bombesin Analogues: Effect of N-terminal vs. Side Chain Iodine Attachment on BBN-GRP Receptor Binding", J. Nucl. Med. 37 (1996) 185P teaches that assessed iodinated BBN analogs as potential SCLC targeting vectors. In all cases, the specific binding region, BBN(8-13) or W-A-V-G-H-L, was maintained, as well as amidation of the carboxy terminal end. Measurement of IC₅₀ values were conducted utilizing Swiss 3T3 cells with [¹²⁵I][Tyr⁴]BBN. The loss of receptor affinity by the mIP-Lys⁷ conjugated peptide suggests that incorporation of Lys between BBN(1-6) may facilitate increased peptide-receptor affinity. The data show that N-terminal iodination of these analogs may provide a viable route to obtain high affinity BBN iodinated peptides.

Schibli, Hoffman. Volkert, et al. "A Tc-99m DITHIA-DI(Bis-Hydroxymethylene) Phosphine conjugate of Bombesin *In Vivo* Studies", J. Nucl. Med. 39 (1998) 225P teaches that the Tc-99 analogs of bombesin derived from 2 different DADT BFCs and the 14 amino acid peptide Lys-3-bombesin were evaluated in a competitive binding assay vs. [¹²⁵I][Tyr⁴]bombesin using human prostate cancer PC-3 cell membranes. The results indicate that the Tc-99m complexes have the potential to be used in the characterization of bombesin/GRP receptors of prostate cancer non-invasively *in vivo.*

Baidoo et al. "Synthesis and Evaluation of High Affinity Technetium Bombesin Analogs", J. Nucl. Med. 38(1997) 87P mentions prostate, breast, gastric, colon, pancreatic and scl cancers. DADT chelates (1 and 2, resulting in neutral or positive cores) were attached to the lysine residue @ N-terminal region of the potent Bn analog Pyr-Q-K-L-G-N-Q-W-A-V-G-H-L-M-NH₂- When a DADT peptide was labeled with Tc-99m or Tc-99, 2 isomers resulted. The Tc-99 analogs exhibited high affinity in a rat cortex membrane binding assay vs. [¹²⁵I][Tyr⁴]bombesin.

B. Rogers, D. Curiel, K. Laffoon, D. Buchsbaum, "Synthesis and Radiolabeling of Bombesin Derivatives with Copper-64 and Binding to Cells Expressing the Gastrin Releasing Peptide Receptor". *Journal of Labelled Compounds and Radiopharmaceuticals* 40 (1997) 482 (abstract) concludes that Cu-64-TETA-Aoc-BBN(7-14) is a potential therapeutic radiopharmaceutical that can be used to treat GRPr positive tumors.

A. Safavy, M. Khazaeli, H. Qin, and D. Buchsbaum, "Synthesis of Bombesin Analogues for Radiolabeling Rhenium-188", *Cancer* 80 (1997) 2354-2359 teaches that 7-amino acid analogue of BBN was synthesized and conjugated to the hydroxamate ligand trisuccin. Radiolabeling with Re-188 were performed in > 90% yield. Cell-binding performed with BNR-11 (3T3 mouse fibroblast cells) and PC-3 human prostate carcinoma GRPA positive cells resulted in positive binding.

B. Rogers et al. "Tumor Localization of a Radiolabeled Bombesin Analog in Mice Bearing Human Ovarian Tumors Induced to Express GRP Receptor by an Adenoviral Vector", *Cancer* 80 (1997) 2419-2424 shows a study was conducted to determine the level of localization of [¹²⁵I/¹³¹I]-mIP-bombesin in tumors.

Rogers, Buchsbaum, et al. "Localization of I-125-mIP-Des-Met14-bombesin(7-13)NH₂ in Ovarian Carcinoma Induced to Express the GRPr by Adenoviral Vector-Mediated Gene Transfer", J. Nucl. Med. *3*8 (1997) 1221-1229 teaches that [¹²⁵I][Tyr⁴]bombesin was compared to [¹²⁵I]-mIP-bombesin (a 7 aa BN analog) for *in vitro* binding and internalization into tumor cells and for tumor localization *in vivo*, and results showed that the latter has more favorable characteristics with regards to tumor localization and cellular internalization & retention.

Zinn, Buchsbaum, et al. "Imaging Adenoviral-Mediated Gene Transfer of GRPr Using a Tc-99m-Labelled Bombesin Analogue", J. Nucl. Med. *3*9 (1998) 224P-225P teaches that BBN analogue (QWAVGHLM) was HYNIC modified and radiolabeled with Tc-99m using tricine as a transchelator. Specific and high affinity binding to GRPr-expressing cells was demonstrated by Scatchard analysis. Favorable biodistribution and imaging were observed.

M.E. Rosenfeld et al., Adenoviral Mediated Delivery of GRPr Results in Specific Tumor Localization of a Bombesin Analogue *In Vivo ", Clin. Cancer Res.* 3 (1997), 1187-1194 teaches similar work to previous publication above.

T.J. Hoffman, G.L. Sieckman and W.A. Volkert. "Targeting Small Cell Lung Cancer Using Iodinated Peptide Analogs" teaches that 5 analogs prepared using SPPS and *in vitro* BB2 receptor binding assessed using Swiss T3T fibroblasts. Amino acids #1-6 nor C-terminal Met residue are not essential to maintain receptor specificity. Results imply that incorporation of I-123 or I-131 as a m-iodophenyl moiety may be used to diagnose or treat sclc.

US 5,686,410 Novartis Albert teaches radiolabeled bombesin and antagonists, including use for tumor imaging and therapy (Examples 11 and 12).

There are numerous articles and patents that discuss the binding of non-radioactive bombesin analogs to various tissues such as SCLC (small cell lung cancer), and petuitary, adrenal, and skin tumors.

### SUMMARY OF THE INVENTION

GRP receptors were found to be overexpressed in prostate cancer, breast cancer, and metastases of such cancers. Several novel isosteric modifications at the N-terminal to enable the introduction of chelating groups and at the third position of the bombesin molecules are introduced. This results in the retention of agonist and internalization properties of the molecules. Radiolabeling of these molecules with imaging and therapeutic isotopes have applications in the detection and treatment of GRP and Bombesin receptor positive tumors.

In one respect, the invention concerns three classes of peptides or peptidomimetics either radiolabeled or not, for the diagnosis and/or therapy of GRP receptor positive tumors.

a. For radiolabeling of bombesin and analogs, the presence of p-Glu at the N-terminal of the peptide chain does not lend itself to the attachment of chelating moieties mentioned below either by conventional means or by solid phase methods.

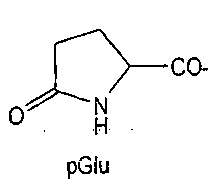

The invention relates to the replacement of the p-Glu moiety with cyclic amino acids without the loss of binding characteristics. Replacement moieties include specifically Proline (Pro) and other cyclic analogs to provide the same tertiary structures the N-terminal. Such a replacement provide a reactive moiety for the attachment polyamino carboxylate chelating groups. NₜS₄₋ₜ chelating agents and others.

Examples of cyclic analogs:

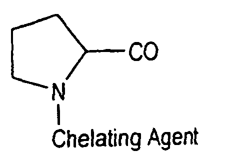

Such an isosteric replacement of pGlu by Pro preserves the binding characteristics of the peptide. Other replacements include pipecolic acid and homologs, and its isomers, and cyclic compounds containing at least one reactive amine to which the chelating agent is attached. 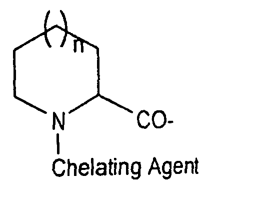 where n = 0 - 5
(Note: n = 0 is Proline, n=1 is pipecolic acid and isomers).

Other cyclic compounds for the replacement of pGlu include the presence of other heteroatom in the carbocyclic ring and the presence of -COOH, NCS (isothiocyanate), NCO (isocyanate), carbomoyl group (OCOX where X is a reactive moiety such as halogen or other reactive moiety). These reactive groups can also be separated from the cyclic moiety by spacer groups. 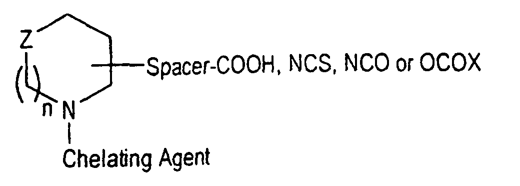 n = 1-6
Z = O, S, N-R (R = alkyl groups C₁-C₆ normal or branched),
Spacer = branched or normal alkyl chain with or without intervening heteroatoms.

The reactive groups for the attachment to the peptide chain indicated can be located at any position of the ring and can be separated by a spacer.

If the attachment of the reactive group is located adjacent to the N- atom containing the chelating moiety or the heteroatom (Z), the attachment can either have either L- or D- configuration.

In all the above the chelating agent is DTPA, DTPA', DOTA, NₜS₄₋ₜ,

b. In another respect, the invention concerns the replacement of the third amino acid of the sequence, Arg, by a chemical equivalent. Such chemical equivalents include, but are not limited to the following. 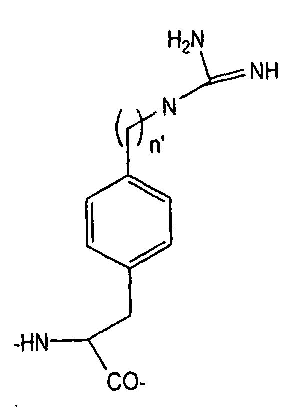 n' = 0-2 (p-gPhe (n'=0), p-gmPhe (n'=1), p-gePhe (n'=2) 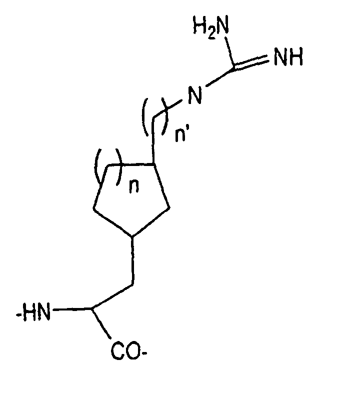 n = 1-3 and n' = 0-3
g-Cpa(n = 1. n' = 0), gm-Cpa (n = 1, n' = 1), ge-Cpa (n = 1, n' = 2), gp-Cpa(n = 1, n'=3)
g-Cha(n = 2, n' = 0), gm-Cha (n = 2, n' = 1), ge-Cha (n = 2, n' = 2), gp-Cha (n = 2, n' = 3)
g-Chpa(n = 2, n' = 0), gm-Chpa (n = 2, n' = 1), ge-Chpa (n = 2, n' = 2), gp-Chpa (n = 2, n' = 3),

c. In another aspect of the invention, pGlu of bombesin is replaced by L- or D-His-AA, or L-His-b-Asp-AA₁ or D-His-Asp-AA₁ (Note: AA₁ is same as in part a, above).
L-(D-)-His-AA₁-Gln-AA₃-AA₄-Gly-Asn-Gln-Trp-Ala-Val-Gly-His-Leu-AA₁₄-NH₂
L-(D-)-His-b-Asp-AA₁-Gln-AA₃-AA₄-Gly-Asn-Gln-Trp-Ala-Val-Gly-His-Leu-AA₁₄-NH₂

d. In another respect the invention relates to, the attachment of radiohalogens. The following compounds can be used without the loss of binding characteristics of bombesin analogs to the receptors.

For all the above compounds, the chelating agent is replaced by o-, m- or p-Iodo(¹²³I, ¹²⁴I, ¹³¹I for imaging) and ¹²⁵I, ¹²⁹I, ¹³¹I for therapy).

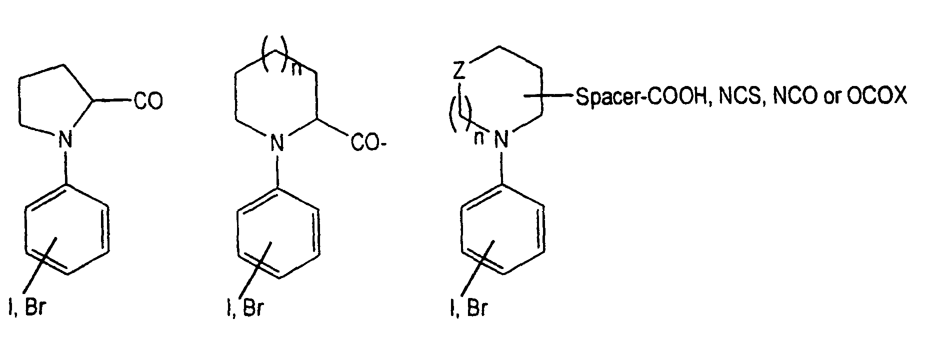

### DETAILED DESCRIPTION OF THE INVENTION

In this specification and claims, numerical values and ranges are not critical unless otherwise stated, that is the numerical values and ranges may be read as if they were prefaced with the word "about" or "substantially".

The invention provides peptides which are analogs of bombesin. By "analogs of bombesin" is meant any compound that binds to a GRP receptor. A particularly preferred analog of bombesin has the formula:

pGlu-Gln-Arg-Tyr-Gly-Asn-Gln-Trp-Ala-Val-Gly-His-Leu-Met-NH₂

This compound is also known as Tyr4-bombesin.

Synthesis of the peptides may be accomplished by well known techniques. Suitable techniques generally involve successive condensations of protected amino acids. Such techniques are well known in the art.

Examples of suitable peptides include the following compounds. The compounds are shown with DTPA an an example of a chelating agent. DTPA can be replaced with DTPA', DOTA, I, Br-Bn, His, His-Asp, etc.
pGlu-Gln-Arg-Tyr-Gly-Asn-Gln-Trp-Ala-Val-Gly-His-Leu-Met-NH₂
DTPA-Pro-Gln-Ara-Tyr-Gly-Asn-Gln-Trp-Ala-Val-Gly-His-Leu-Met-NH₂
DTPA-Pip-Gln-Arg-Tyr-Gly-Asn-Gln-Trp-Ala-Val-Gly-His-Leu-Met-NH₂ (Pip = pipecolic acid)
DTPA-hPip-Gln-Arg-Tyr-Gly-Asn-Gln-Trp-Ala-Val-Gly-His-Leu-Met-NH₂ (Pip = homopipecolic acid)
DTPA-Pro-Gln-Arg-Tyr-Gly-Asn-Gln-Trp-Ala-Val-Gly-His-Leu-Nle-NH₂
DTPA-Pip-Gln-Arg-Tyr-Gly-Asn-Gln-Trp-Ala-Val-Gly-His-Leu-Nle-NH₂ (Pip = pipecolic acid)
DTPA-hPip-Gln-Arg-Tyr-Gly-Asn-Gln-Trp-Ala-Val-Gly-His-Leu-Nle-NH₂ (Pip = homopipecolic acid)
DTPA-Moc-Gln-Arg-Tyr-Gly-Asn-Gln-Trp-Ala-Val-Gly-His-Leu-Met-NH₂ (Moc = morpholino 2-carboxylic acid)
DTPA-Mtc-Gln-Arg-Tyr-Gly-Asn-Gln-Trp-Ala-Val-Gly-His-Leu-Met-NH₂ (Moc = thiomorpholino 2-carboxylic acid)
DTPA-Pro-Gln-gPhe-Tyr-Gly-Asn-Gln-Trp-Ala-Val-Gly-His-Leu-Met-NH₂
DTPA-Pro-Gln-gmPhe-Tyr-Gly-Asn-Gln-Trp-Ala-Val-Gly-His-Leu-Met-NH₂
DTPA-Pro-Gln-gePhe-Tyr-Gly-Asn-Gln-Trp-Ala-Val-Gly-His-Leu-Met-NH₂
DTPA-Pip-Gln-gPhe-Tyr-Gly-Asn-Gln-Trp-Ala-Val-Gly-His-Leu-Met-NH₂
DTPA-Pip-Gln-gmPhe-Tyr-Gly-Asn-Gln-Trp-Ala-Val-Gly-His-Leu-Met-NH₂
DTPA-Pip-Gln-gePhe-Tyr-Gly-Asn-Gln-Trp-Ala-Val-Gly-His-Leu-Met-NH₂
DTPA-hPip-Gln-gPhe-Tyr-Gly-Asn-Gln-Trp-Ala-Val-Gly-His-Leu-Met-NH₂
DTPA-Moc-Gln-gmPhe-Tyr-Gly-Asn-Gln-Trp-Ala-Val-Gly-His-Leu-Met-NH₂
DTPA-Mtc-Gln-gePhe-Tyr-Gly-Asn-Gln-Trp-Ala-Val-Gly-His-Leu-Met-NH₂
DTPA-Pro-Gln-gCpa-Tyr-Gly-Asn-Gln-Trp-Ala-Val-Gly-His-Leu-Met-NH₂
DTPA-Pro-Gln-gmCpa-Tyr-Gly-Asn-Gln-Trp-Ala-Val-Gly-His-Leu-Met-NH₂
DTPA-Pro-Gln-geCpa-Tyr-Gly-Asn-Gln-Trp-Ala-Val-Gly-His-Leu-Met-NH₂
DTPA-Pro-Gln-gpCpa-Tyr-Gly-Asn-Gln-Trp-Ala-Val-Gly-His-Leu-Met-NH₂
DTPA-Pro-Gln-gCha-Tyr-Gly-Asn-Gln-Trp-Ala-Val-Gly-His-Leu-Met-NH₂
DTPA-Pro-Gln-gmCha-Tyr-Gly-Asn-Gln-Trp-Ala-Val-Gly-His-Leu-Met-NH₂
DTPA-Pro-Gln-geCha-Tyr-Gly-Asn-Gln-Trp-Ala-Val-Gly-His-Leu-Met-NH₂
DTPA-Pro-Gln-gpCha-Tyr-Gly-Asn-Gln-Trp-Ala-Val-Gly-His-Leu-Met-NH₂
DTPA-Pro-Gln-gChpa-Tyr-Gly-Asn-Gln-Trp-Ala-Val-Gly-His-Leu-Met-NH₂
DTPA-Pro-Gln-gmChpa-Tyr-Gly-Asn-Gln-Trp-Ala-Val-Gly-His-Leu-Met-NH₂
DTPA-Pro-Gln-geChpa-Tyr-Gly-Asn-Gln-Trp-Ala-Val-Gly-His-Leu-Met-NH₂
DTPA-Pro-Gln-gpChpa-Tyr-Gly-Asn-Gln-Trp-Ala-Val-Gly-His-Leu-Met-NH₂
DTPA-Pip-Gln-gCpa-Tyr-Gly-Asn-Gln-Trp-Ala-Val-Gly-His-Leu-Met-NH₂
DTPA-Pip-Gln-gmCpa-Tyr-Gly-Asn-Gln-Trp-Ala-Val-Gly-His-Leu-Met-NH₂
DTPA-Pip-Gln-geCpa-Tyr-Gly-Asn-Gln-Trp-Ala-Val-Gly-His-Leu-Met-NH₂
DTPA-Pip-Gln-gpCpa-Tyr-Gly-Asn-Gln-Trp-Ala-Val-Gly-His-Leu-Met-NH₂
DTPA-Pip-Gln-gCha-Tyr-Gly-Asn-Gln-Trp-Ala-Val-Gly-His-Leu-Met-NH₂
DTPA-Pip-Gln-gmCha-Tyr-Gly-Asn-Gln-Trp-Ala-Val-Gly-His-Leu-Met-NH₂
DTPA-Pip-Gln-geCha-Tyr-Gly-Asn-Gln-Trp-Ala-Val-Gly-His-Leu-Met-NH₂
DTPA-Pip-Gln-gpCha-Tyr-Gly-Asn-Gln-Trp-Ala-Val-Gly-His-Leu-Met-NH₂
DTPA-Pip-Gln-gChpa-Tyr-Gly-Asn-Gln-Trp-Ala-Val-Gly-His-Leu-Met-NH₂
DTPA-Pip-Gln-gmChpa-Tyr-Gly-Asn-Gln-Trp-Ala-Val-Gly-His-Leu-Met-NH₂
DTPA-Pip-Gln-geChpa-Tyr-Gly-Asn-Gln-Trp-Ala-Val-Gly-His-Leu-Met-NH₂
DTPA-Pip-Gln-gpChpa-Tyr-Gly-Asn-Gln-Trp-Ala-Val-Gly-His-Leu-Met-NH₂
DTPA-hPip-Gln-gCpa-Tyr-Gly-Asn-Gln-Trp-Ala-Val-Gly-His-Leu-Met-NH₂
DTPA-hPip-Gln-gmCpa-Tyr-Gly-Asn-Gln-Trp-Ala-Val-Gly-His-Leu-Met-NH₂
DTPA-hPip-Gln-geCpa-Tyr-Gly-Asn-Gln-Trp-Ala-Val-Gly-His-Leu-Met-NH₂
DTPA-hPip-Gln-gpCpa-Tyr-Gly-Asn-Gln-Trp-Ala-Val-Gly-His-Leu-Met-NH₂
DTPA-hPip-Gln-gCha-Tyr-Gly-Asn-Gln-Trp-Ala-Val-Gly-His-Leu-Met-NH₂
DTPA-hPip-Gln-gmCha-Tyr-Gly-Asn-Gln-Trp-Ala-Val-Gly-His-Leu-Met-NH₂
DTPA-hPip-Gln-geCha-Tyr-Gly-Asn-Gln-Trp-Ala-Val-Gly-His-Leu-Met-NH₂
DTPA-hPip-Gln-gpCha-Tyr-Gly-Asn-Gln-Trp-Ala-Val-Gly-His-Leu-Met-NH₂
DTPA-hPip-Gln-gChpa-Tyr-Gly-Asn-Gln-Trp-Ala-Val-Gly-His-Leu-Met-NH₂
DTPA-hPip-Gln-gmChpa-Tyr-Gly-Asn-Gln-Trp-Ala-Val-Gly-His-Leu-Met-NH₂
DTPA-hPip-Gln-geChpa-Tyr-Gly-Asn-Gln-Trp-Ala-Val-Gly-His-Leu-Met-NH₂
DTPA-hPip-Gln-gpChpa-Tyr-Gly-Asn-Gln-Trp-Ala-Val-Gly-His-Leu-Met-NH₂
DTPA-Moc-Gln-gCpa-Tyr-Gly-Asn-Gln-Trp-Ala-Val-Gly-His-Leu-Met-NH₂
DTPA-Moc-Gin-gmCpa-Tyr-Gly-Asn-Gin-Trp-Ala-Val-Gly-His-Leu-Met-NH₂
DTPA-Moc-Gln-geCpa-Tyr-Gly-Asn-Gln-Trp-Ala-Val-Gly-His-Leu-Met-NH₂
DTPA-Moc-Gln-gpCpa-Tyr-Gly-Asn-Gln-Trp-Ala-Val-Gly-His-Leu-Met-NH₂
DTPA-Moc-Gln-gCha-Tyr-Gly-Asn-Gln-Trp-Ala-Val-Gly-His-Leu-Met-NH₂
DTPA-Moc-Gln-gmCha-Tyr-Gly-Asn-Gln-Trp-Ala-Val-Gly-His-Leu-Met-NH₂
DTPA-Moc-Gln-geCha-Tyr-Gly-Asn-Gln-Trp-Ala-Val-Gly-His-Leu-Met-NH₂
DTPA-Moc-Gln-gpCha-Tyr-Gly-Asn-Gln-Trp-Ala-Val-Gly-His-Leu-Met-NH₂
DTPA-Moc-Gln-gChpa-Tyr-Gly-Asn-Gln-Trp-Ala-Vat-Gly-His-Leu-Met-NH₂
DTPA-Moc-Gln-gmChpa-Tyr-Gly-Asn-Gln-Trp-Ala-Val-Gly-His-Leu-Met-NH₂
DTPA-Moc-Gln-geChpa-Tyr-Gly-Asn-Gln-Trp-Ala-Val-Gly-His-Leu-Met-NH₂
DTPA-Moc-Gln-gpChpa-Tyr-Gly-Asn-Gln-Trp-Ala-Val-Gly-His-Leu-Met-NH₂
DTPA-Mtc-Gln-gCpa-Tyr-Gly-Asn-Gln-Trp-Ala-Val-Gly-His-Leu-Met-NH₂
DTPA-Mtc-Gln-gmCpa-Tyr-Gly-Asn-Gln-Trp-Ala-Val-Gly-His-Leu-Met-NH₂
DTPA-Mtc-Gln-geCpa-Tyr-Gly-Asn-Gln-Trp-Ala-Val-Gly-His-Leu-Met-NH₂
DTPA-Mtc-Gln-gpCpa-Tyr-Gly-Asn-Gln-Trp-Ala-Val-Gly-His-Leu-Met-NH₂
DTPA-Mtc-Gln-gCha-Tyr-Gly-Asn-Gln-Trp-Ala-Val-Gly-His-Leu-Met-NH₂
DTPA-Mte-Gln-gmCha-Tyr-Gly-Asn-Gln-Trp-Ala-Val-Gly-His-Leu-Met-NH₂
DTPA-Mtc-Gln-geCha-Tyr-Gly-Asn-Gln-Trp-Ala-Val-Gly-His-Leu-Met-NH₂
DTPA-Mtc-Gln-gpCha-Tyr-Gly-Asn-Gln-Trp-Ala-Val-Gly-His-Leu-Met-NH₂
DTPA-Mtc-Gln-gChpa-Tyr-Gly-Asn-Gln-Trp-Ala-Val-Gly-His-Leu-Met-NH₂
DTPA-Moc-Gln-gmChpa-Tyr-Gly-Asn-Gln-Trp-Ala-Val-Gly-His-Leu-Met-NH₂
DTPA-Moc-Gln-geChpa-Tyr-Gly-Asn-Gln-Trp-Ala-Val-Gly-His-Leu-Met-NH₂
DTPA-Moc-Gln-gpChpa-Tyr-Gly-Asn-Gln-Trp-Ala-Val-Gly-His-Leu-Met-NH₂

The peptides are bound to a suitable radionuclide. For diagnosis, suitable radionuclides include ^{133m}In, ^{99m}Tc, ⁶⁷Ga, ⁶⁸Ga ⁷²As, ¹¹¹In, ⁹⁷Ru, ²⁰³Pb, ⁶²Cu, ⁶⁴Cu, ⁵²Fe, ^{52m}Mn, ⁵¹Cr, ¹⁵⁷Gd, ¹²³I, ¹²⁴I, ¹³¹I, ⁷⁵Br, ⁷⁶Br, ⁷⁷Br, and ⁸²Br with ^{99m}Tc, ⁶⁷Ga, ¹¹¹In, and ¹²³I being preferred. For therapy, suitable radionuclides include ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁹⁹Au, ¹⁶¹Tb, ¹⁰⁹Pd, ¹⁶⁶Dy, ¹⁶⁶Ho, ¹⁴⁹Pm, ¹⁵¹Pm, ¹⁵³Sm, ¹⁵⁹Gd, ¹⁷²Tm, ¹⁶⁹Yb, ¹⁷⁵Yb, ¹⁷⁷Lu, ¹⁰⁵Rh, ¹¹¹Ag, ¹³¹I, ¹²⁹I, and ^{177m}Sn, with ¹⁸⁶Re, ¹⁸⁸Re, ⁹⁰Y, ¹⁵³Sm, ¹⁷⁷Lu, and ¹³¹I being preferred.

The radionuclide and the peptide must be bound together. If the radionuclide is a radioactive halogen, the radioactive halogen may be bound directly to the peptide, such as by chemical reaction to a Tyr or Trp moiety of the peptide.

If the radionuclide is a radioactive metal, the radioactive metal may be bound to the peptide by means of a chelating agent. A chelating group may be attached to the peptide by an amide bond or through a spacing group.

Suitable chelating groups for chelating said metal atoms are NₜS₍₄₋ₜ₎ tetradentate chelating agents, wherein t=2-4, or groups derived from ethylene diamine tetra-acetic acid (EDTA), diethylene triamine penta-acetic acid (DTPA), cyclohexyl 1,2-diamine tetra-acetic acid (CDTA), ethyleneglycol-0,0'-bis(2-aminoethyl)-N,N,N',N'-tetra-acetic acid (EGTA), N,N-bis(hydroxybenzyl)-ethylenediamine-N,N'-diacetic acid (HBED), triethylene tetramine hexa-acetic acid (TTHA), 1,4,7,10-tetraazacyclododecane-N,N',N",N"'-tetra-acetic acid (DOTA), hydroxyethyldiamine triacetic acid (HEDTA), 1,4,8,11-tetra-azacyclotetradecane-N,N',N',N"'-tetra-acetic acid (TETA), substituted DTPA, substituted EDTA, or from a compound of the general formula 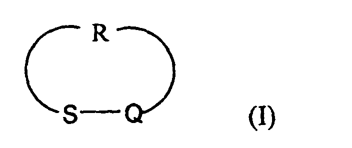 wherein R is a branched or non-branched, optionally substituted hydrocarbyl radical, which may be interrupted by one or more hetero-atoms selected from N, O and S and/or by one or more NH groups, and Q is a group which is capable of reacting with an amino group of the peptide and which is preferably selected from the group consisting of carbonyl, carbimidoyl, N-(C₁-C₆)alkylcarbimidoyl, N-hydroxycarbimidoyl and N-(C₁-C₆)alkoxycarbimidoyl.

NₜS₍₄₋ₜ₎ chelating agents, wherein t = 2-4, are preferably selected from 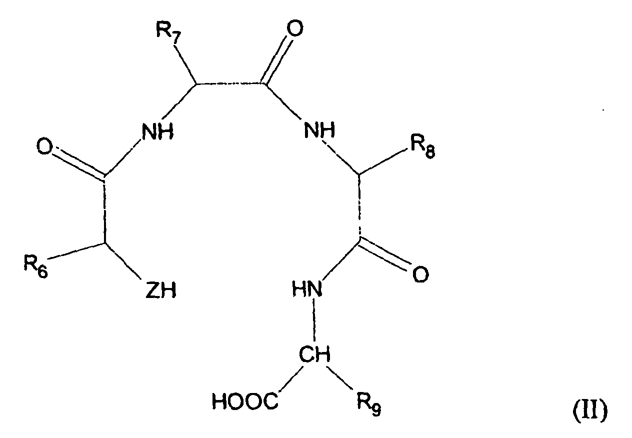 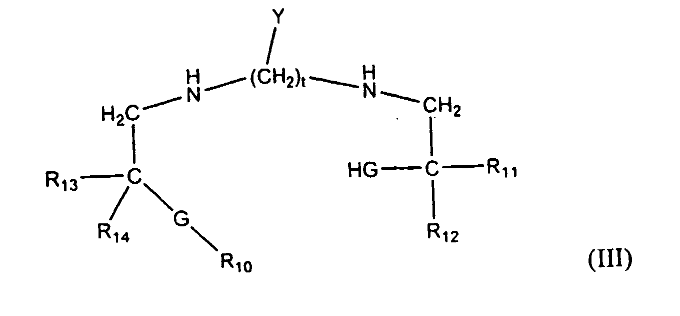 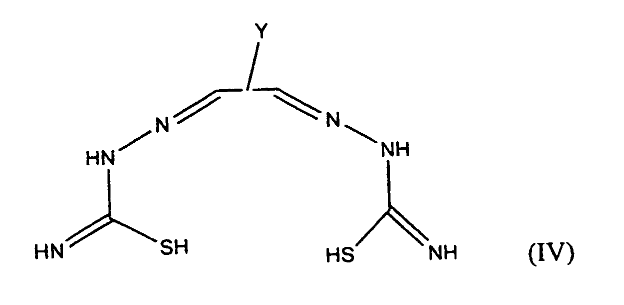 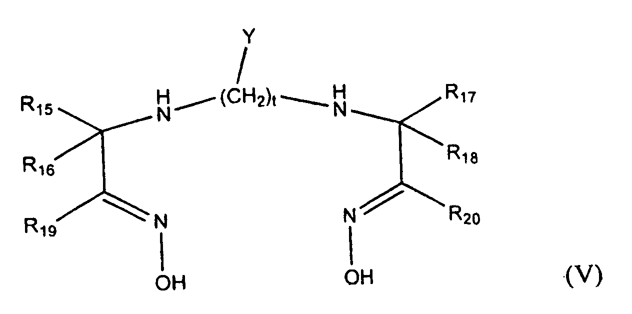 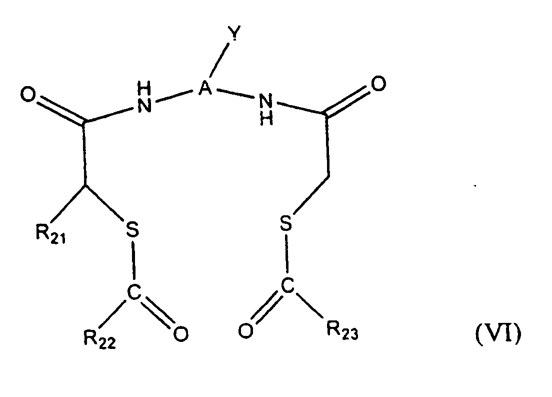 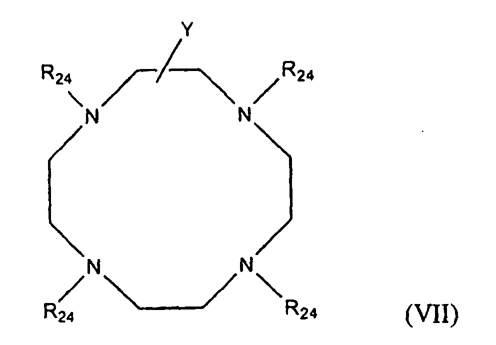 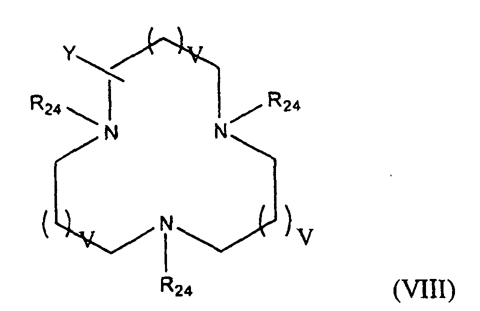 wherein:
R₆-R₂₀ are each individually hydrogen atoms or (C₁-C₄)alkyl groups, with the proviso that at least one of C₆ to C₉ is the symbol Y;
R₂₁ is a hydrogen atom or a CO₂(C₁-C₄)alkyl group;
R₂₂ and R₂₃ are each individually (C₁-C₄)alkyl groups or phenyl groups;
v is 0 or 1;
t is 2 or 3;
R₂₄ is CH₂COOH or a functional derivative thereof;
A is (C₁-C₄)alkylene, if desired substituted with CO₂alkyl, CH₂COalkyl, CONH₂, CONHCH₂CO₂alkyl; phenylene, phenylene substituted by CO₂alkyl, wherein the alkyl groups have 1 to 4 carbon atoms;
G is NH or S;
Y is a functional group capable of binding with a free amino group of the peptide or with the spacing group;
and Z is S or O.

Said functional group Y preferably comprises isocyanato, isothiocyanato, formyl, o-halonitrophenyl, diazonium, epoxy, trichloro-s-triazinyl, ethyleneimino, chlorosulfonyl, alkoxycarb-imidoyl, (substituted or unsubstituted) alkylcarbonyloxycarbonyl, alkylcarbonylimidazolyl, succinimido-oxycarbonyl; said group being attached to a (C₁-C₁₀)hydrocarbon biradical. Suitable examples of hydrocarbon biradicals are biradicals derived from benzene, (C₁-C₆)alkanes, (C₂-C₆)alkenes and (C₁-C₄)-alkylbenzenes.

Examples of suitable chelators of the general formula II are described in the international patent application WO 89/07456, such as unsubstituted or substituted 2-imino-thiolanes and 2-imino-thiacyclohexanes, in particular 2-imino-4-mercaptomethylthiolane.

Suitable examples of spacing groups, if present in the metal-labelled peptide molecule, are groups of the general formula 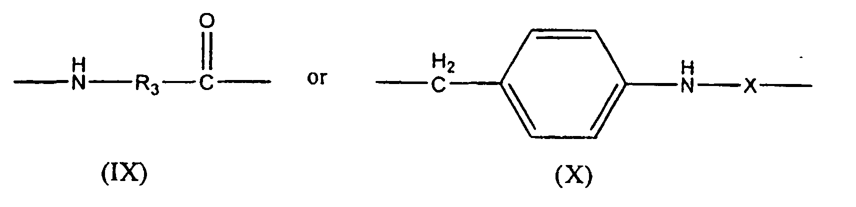 wherein R₃ is a C₁-C₁₀ alkylene group, a C₁-C₁₀ alkylidene group or a C₂-C₁₀ alkenylene group, and X is a thiocarbonyl group or a group of the general formula 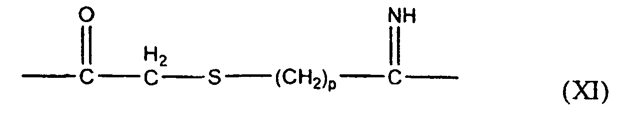 wherein p is 1-5.

Conjugates with avidin or biotin are formed as described by Paganelli et al. (Int. J. Cancer 1988, 2, 121). Kalofonos et al. (J. Nucl. Med. 1990, 31, 1791) and Anderson et al. (FEBS LETT. 1991, 282/1, 35-40).

The labeled peptides may be combined with carrier materials, such as saline, and adjuvants, such as acids or bases added to change the pH, buffers, and preservatives. The use of carriers and adjuvants is well known to those skilled in the art.

The invention may be provided to the user by providing a suitable radiolabeled peptide of the invention in a carrier, with or without adjuvants, or by providing some or all of the necessary components in a kit. The use of a kit is particularly convenient since some of the components have a limited shelf life, particularly when combined. A suitable kit may include one or more of the following components (i) a peptide, (ii) a chelating agent, (iii) a carrier solution, (iv) a radioisotope, (v) a reducing agent, and (vi) instructions for their combination. Depending on the form of the radionuclide, the reducing agent be a necessary to prepare the radionuclide for reaction with the peptide. Suitable reducing agents include Ce(III), Fe(II), Cu(I), Ti(III), Sb(III), and Sn(II). Of these. Sn(II) is particularly preferred.

For reasons of stability, it is generally preferred that the peptide be in a dry, lyophilized condition. The user can add the carrier solution to the dry peptide to reconstitute it. If it is desired to provide the peptide in solution form, it may be necessary to store it at lower temperatures than the dry form.

As mentioned above, the peptide and the chelating agent may be included separately in the kit. Alternatively, the peptide may have already been combined with the chelating agent.

Because of the short half-life of suitable radionuclides, it will frequently be most convenient to provide the kit without the radionuclide to the user, who will order the radionuclide separately when needed for a procedure. If the radionuclide is included in the kit, the kit will most likely be shipped to the user just before it is needed.

For diagnosis of tumors, the radiolabeled compounds are administered in an amount effective to allow imaging of the tumors. The quantitative amount will vary depending on the uptake of the compound by the tumor, the radioisotope chosen, and the sensitivity of the detection device (e.g.: gamma camera). Too little compound will not allow a sufficient radiation to permit diagnosis. Too much compound will cause large concentrations of the compound in the blood or non-targeted organs, and may also present unnecessary risk of toxicity to the patient. The selection of the effective amount is within the skill of one skilled in the art.

For treatment of tumors, the radiolabeled compounds are administered in a therapeutically effective amount. By "therapeutically effective amount" is meant an amount that will at least inhibit the growth or spread of the tumor, and preferably will cause the tumor to shrink or be completely eliminated. The quantitative amount will vary depending on the uptake of the compound by the tumor and the radioisotope chosen. Too little compound will not have sufficient effect. Too much compound will present unnecessary radiation exposure and risk of toxicity to the patient. The selection of the effective amount is within the skill a typical radiation oncologist.

### EXAMPLE 1

The peptide

pGlu-Gln-Arg-Tyr-Gly-Asn-Gln-Trp-Ala-Val-Gly-His-Leu-Met-NH₂

([Tyr⁴]bombesin) was synthesized using conventional solid phase techniques. It was labeled with ¹²⁵I by the chloramine T iodination procedure, according to Greenwood, et al., Biochemical Journal 1963, 89, 114-123. The resulting compound

pGlu-Gln-Arg-Tyr*-Gly-Asn-Gln-Trp-Ala-Val-Gly-His-Leu-Met-NH₂

([¹²⁵I-Tyr⁴]bombesin, the "radioligand") was purified by HPLC, and had a specific activity of 1,000 Ci/mmol.

### EXAMPLE 2

Various tumor tissue samples, including surrounding tissue, were harvested from human patients and/or human cadavers, and frozen. The samples were cut on a cryostat, mounted on microscope slides, and then stored at -20°C for at least 3 days to at least 3 days to improve adhesion of the tissue to the slide. The slide-mounted tissue sections were allowed to reach room temperature and preincubated in 50 mmol/l Tris-HCl, 130 mmol/l NaCl, 4.7 mmol/l KCl, 5 mmol/l MgCl₂, 1 mmol/l ethylene glycol-bi(b-aminoethylether)-N,N,N',N'-tetraacetic acid, and 0.5% bovine serum albumin, pH 7.4 (preincubation solution), for 30 min. at 25°C. The slides are then incubated in a solution containing the same medium as the preincubation solution except the bovine serum albumin is omitted, and the following compounds are added: 20000 dpm/100 ml of the radioligand of Example 1, 0.025% bacitracin, 1 mmol/l dithiothreitol, 2mg/ml chymostatin, and 4mg/ml leupeptin, pH = 6.5. The slides are incubated at room temperature with the radioligand for 150 min., as described by Mantyh et al. (Gasteroenterology 1994, 107, 1019-30). After the incubation, the slides are rinsed with four washes of 30 sec each in ice-cold preincubation solution, pH 7.4, dipped in ice-cold water, and then quickly dried in a refrigerator under a stream of cold air. The sections are subsequently exposed to a ³H-Ultrofilm for 1 week, to detect the precise location of the radioactivity.

The films were evaluated to determine the ability to distinguish the tumor from the surrounding tissue. The results are shown in Table 1.

**TABLE 1**

| Tissue | Number of Samples | Number Positive | % Positive |
|---|---|---|---|
| Colon Cancer | 18 | 1 | 6 |
| Gastric Cancer | 27 | 0 | 0 |
| Pancreatic Cancer | 28 | 0 | 0 |
| Non Small Cell Lung Carcinoma | 34 | 0 | 0 |
| Small Cell Lung Carcinoma | 10 | 2 | 20 |
| Gastrinomas | 4 | 4 | 100 |
| Melanomas | 8 | 1 | 12 |
| Glioblastomas | 9 | 6 | 67 |
| Prostate Cancer | 28 | 28 | 100 |
| Prostate Cancer Metastases | 5 | 5 | 100 |
| Breast Cancer | 95 | 66 | 69 |
| Breast Cancer Metastases | 5 | 5 | 100 |
| Uterus Leiomyosarcoma Tumor | 3 | 1 | 33 |

In Table 1, a tumor is considered "positive" if (1) a traditional histilogical examination of the tissue verifies that the tumor is present, (2) the film shows a clear image of the tumor, distinguishing it from the surrounding tissue, and (3) the film does not show an image of the tumor, if the tissue is first blocked with a nonradiolabeled sample of the peptide.

The data show that (1) gastrinomas, glioblastomas, prostate cancer, prostate cancer metastases, breast cancer, and breast cancer metastases show a high incidence of bombesin receptors (in the case of gastrinomas, prostate cancer, prostate cancer metastases, and breast cancer metastases, 100%); and (2) a number of human tumors that were claimed by the literature to be bombesin receptor positive show no or very little positive results.

The negative data in Table 1 is important in understanding the instant invention. It has been widely reported (see, for example, Moody et al., Peptides, 683-686 (1993)), that small cell lung cancer has bombesin receptors. However, the data in Table 1 shows that only 20% of such tumors could be detected. Because of the highly specific nature of the method used to produce the data in Table 1, the data suggests that the techniques used in the prior art had fundamental defects.

### EXAMPLE 3

The peptide

DTPA-Pro-Gln-Arg-Tyr-Gly-Asn-Gln-Trp-Ala-Val-Gly-His-Leu-Met-NH₂

[DTPA⁰-Pro¹,Tyr⁴]bombesin) was synthesized using conventional solid phase techniques. DTPA was introduced during the solid phase synthesis using tri-t-butyl DTPA. ¹¹¹In labeling was performed according to the procedure described by W.H. Bakker, et al., Life Sciences, Vol 49, 1583 (1991), yielding [¹¹¹In-DTPA⁰-Pro¹,Tyr⁴]bombesin having a specific activity of 100 MBq/µg.

### EXAMPLE 4

Gastrin releasing peptide receptor has high affinity for the 14 amino acid peptide bombesin. A bombesin analog, [¹¹¹In-DTPA⁰,Pro¹,Tyr⁴]bombesin, showed intact high affinity to the bombesin receptor, and agonistic activities on bombesin-stimulated prolactin secretion on 7315b cells with an IC50 of 8 nM. After labeling with IN-111 up to a level of 100 Mbq per µg, the radioligand was radiochemically stable (>95%) for 2 h, as revealed by HPLC. In rats high and specific uptake was found in pancreas and tissues of the GI tract. Uptake of radioactivity could be blocked by iv coinjection of 100 µg Tyr⁴bombesin with the radioligand, but not when administered 1 h after the radioligand, indicating its intemalisation. A bell-shaped function between injected mass and % ID per g bombesin receptor positive tissues was found at ≈ 0.025-0.1 µg. Dynamic gamma camera showed rapid clearance of radioactivity from the blood compartment, renal uptake and urinary excretion: ≈ 35% in 1 h, 70% in 20 h with a total body retention of 10%. Specific uptake in the bombesin receptor positive prolactinoma 7315b inoculated on female Lewis rats was found and could also be visualized by scintigraphy. The residence time of radioactivity was in accordance with similar DTPA conjugated peptides. Thus, the radioligand [¹¹¹In-DTPA⁰,Pro¹,Tyr⁴]bombesin is suitable for scintigraphy of bombesin receptors in vivo.

**TABLE 2**

| Tissue RA in % ID/g at 48 h after injection of 0.01-0.05 µg of [¹¹¹In-DTPA⁰,Pro¹,Tyr⁴]bombesin labeled with 2 MBq In-111 in rats (n≥3) and ratio vs. blood( ) | | | | | |
|---|---|---|---|---|---|
| µg | Pancreas | Colon | Stomach | Adrenal | Blood |
| 0.01 | 1.0(1210) | 0.072(85) | 0.035(40) | 0.029(34) | 0.0009 |
| 0.025 | 1.2(1217) | 0.063(64) | 0.065(64) | 0.030(30) | 0.0010 |
| 0.1 | 0.72(856) | 0.061(73) | 0.042(50) | 0.022(26) | 0.0009 |
| 0.5 | 0.43(574) | 0.039(52) | 0.036(50) | 0.014(18) | 0.0008 |

## Claims

1. A peptide AA₁-Gln-AA₃-Leu-Gly-Asn-Gln-Trp-Ala-Val-Gly-His-Leu-Met-NH₂,
wherein AA₁ is a cyclic amino acid of the formula 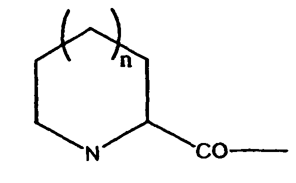 wherein n = 0 to 5; and
wherein AA₃ is selected from the group consisting of Arg, 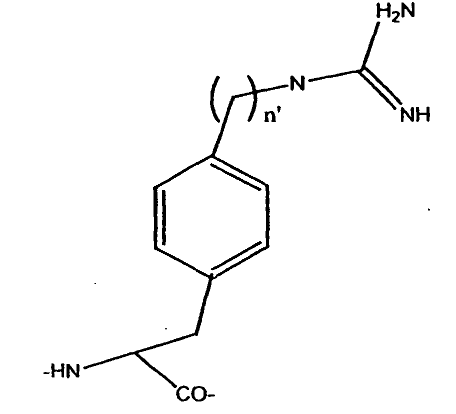 wherein n' = 0 to 2, and 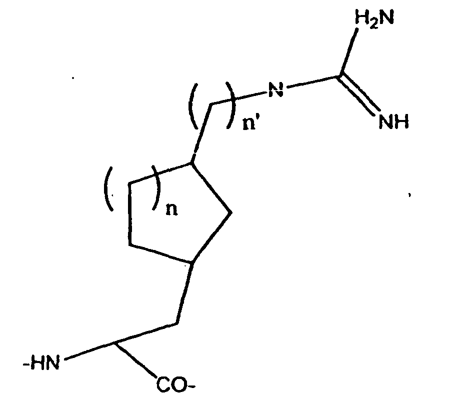 wherein n = 1 to 3 and n' = 0 to 3.

2. The peptide according to Claim 1 wherein AA₁ is selected from the group consisting of Pro, Pip, hPip, Moc and Mtc, and AA₃ is selected from the group consisting of Arg, gPhe, gmPhe, gePhe, gCpa, gmCpa, gpCpa, gCha, gmCha, geCha, gpCha, gChpa, gmChpa, geChpa and gpChpa.

3. The peptide according to Claim 1 wherein AA₁ is selected from the group consisting of L-His-AA₁, D-His-AA₁, L-His-Asp-AA₁ and D-His-Asp-AA₁.

4. The peptide according to Claim 1 wherein the cyclic ring further includes a heteroatom selected from the group consisting of O, S and N-R, wherein R = alkyl groups C₁-C₆ normal or branched.

5. The peptide according to Claim 1 wherein the -CO- group is replaced by a branched or normal alkyl chain with or without intervening heteroatoms and a reactive group selected from the group consisting of COOH, NCS, NCO and OCOX, wherein X is a reactive moiety.

6. The peptide according to Claim 1 further including a diagnostic or therapeutic radionuclide coupled to the peptide by a chelating agent.

7. The peptide according to Claim 6 wherein the diagnostic radionuclide is selected from the group consisting of ^{133m}In, ^{99m}Tc, ⁶⁷Ga, ⁶⁸Ga, ⁷²As, ¹¹¹In, ⁹⁷Ru, ²⁰³Pb, ⁶²Cu, ⁶⁴Cu, ^{133m}In, ⁵¹Cr, ¹⁵⁷Gd, ¹²³I, ¹²⁴I, ¹³¹I, ⁷⁵Br, ⁷⁶Br, ⁷⁷Br and ⁸²Br.

8. The peptide according to Claim 6 wherein the therapeutic radionuclide is selected from the group consisting of ¹⁸⁶Re, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁹⁹Au, ¹⁶¹Tb, ¹⁰⁹Pd, ¹⁸⁸Re, ⁷⁷As, ¹⁶⁶Dy, ¹⁶⁶Ho, ¹⁴⁹Pm, ¹⁵¹Pm, ¹⁵³Sm, ¹⁵⁹Gd, ¹⁷²Tm, ¹⁶⁹Yb, ¹⁷⁵Yb, ¹⁷⁷Lu, ¹⁰⁵Rh, ¹¹¹Ag, ¹³¹I, ¹²⁹I and ^{177m}Sn.

9. The peptide according to Claim 6 wherein the chelating agent is selected from the group consisting of DTPA, DTPA', DOTA and NₜS₄₋ₜ.

10. A pharmaceutical composition comprising the labeled peptide of Claims 6-9 and a carrier material or adjuvant.

11. A kit for the diagnosis of breast or prostate tumors or metastases of such tumors in a human comprising
(a) a peptide according to claim 1;
(b) a radioisotope; and
(c) adjuvants suitable for binding the radioisotope to the peptide and administering the resultant combination to a human.

12. A peptide according to claim 1 for use in diagnosis.

13. Use of a peptide according to claim 1 in the manufacture of a medicament for the diagnosis of GRP receptor positive tumors.

14. Use of a peptide according to claim 1 in the manufacture of a medicament for the diagnosis of prostate cancer, breast cancer and metastases of such cancers.

15. A kit for the treatment of breast or prostate tumors or metastases of such tumors in a human comprising
(a) a peptide according to claim 1;
(b) a radioisotope; and
(c) adjuvants suitable for binding the radioisotope to the peptide and administering the resultant combination to a human.

16. A peptide according to claim 1 for use in therapy.

17. Use of a peptide according to claim 1 in the manufacture of a medicament for the treatment of GRP receptor positive tumors.

18. Use of a peptide according to claim 1 in the manufacture of a medicament for the treatment of prostate cancer, breast cancer and metastases of such cancers.

19. A peptide selected from the group consisting of
DTPA-Pip-Gln-Arg-Tyr-Gly-Asn-Gln-Trp-Ala-Val-Gly-His-Leu-Met-NH_{2,}
DTPA-hPip-Gln-Arg-Tyr-Gly-Asn-Gln-Trp-Ala-Val-Gly-His-Leu-Met-NH_{2,}
DTPA-Pro-Gln-Arg-Tyr-Gly-Asn-Gln-Trp-Ala-Val-Gly-His-Leu-Nle-NH₂,
DTPA-Pip-Gln-Arg-Tyr-Gly-Asn-Gln-Trp-Ala-Val-Gly-His-Leu-Nle-NH₂,
DTPA-hPip-Gln-Arg-Tyr-Gly-Asn-Gln-Trp-Ala-Val-Gly-His-Leu-Nle-NH_{2,}
DTPA-Moc-Gln-Arg-Tyr-Gly-Asn-Gln-Trp-Ala-Val-Gly-His-Leu-Met-NH_{2,}
DTPA-Mtc-Gln-Arg-Tyr-Gly-Asn-Gln-Trp-Ala-Val-Gly-His-Leu-Met-NH_{2,}
DTPA-Pro-Gln-gPhe-Tyr-Gly-Asn-Gln-Trp-Ala-Val-Gly-His-Leu-Met-NH_{2,}
DTPA-Pro-Gln-gmPhe-Tyr-Gly-Asn-Gln-Trp-Ala-Val-Gly-His-Leu-Met-NH_{2,}
DTPA-Pro-Gln-gePhe-Tyr-Gly-Asn-Gln-Trp-Ala-Val-Gly-His-Leu-Met-NH_{2,}
DTPA-Pip-Gln-gPhe-Tyr-Gly-Asn-Gln-Trp-Ala-Val-Gly-His-Leu-Met-NH_{2,}
DTPA-Pip-Gln-gmPhe-Tyr-Gly-Asn-Gln-Trp-Ala-Val-Gly-His-Leu-Met-NH_{2,}
DTPA-Pip-Gln-gePhe-Tyr-Gly-Asn-Gln-Trp-Ala-Val-Gly-His-Leu-Met-NH_{2,}
DTPA-hPip-Gln-gPhe-Tyr-Gly-Asn-Gln-Trp-Ala-Val-Gly-His-Leu-Met-NH_{2,}
DTPA-Moc-Gln-gmPhe-Tyr-Gly-Asn-Gln-Trp-Ala-Val-Gly-His-Leu-Met-NH_{2,}
DTPA-Mtc-Gln-gePhe-Tyr-Gly-Asn-Gln-Trp-Ala-Val-Gly-His-Leu-Met-NH_{2,}
DTPA-Pro-Gln-gCpa-Tyr-Gly-Asn-Gln-Trp-Ala-Val-Gly-His-Leu-Met-NH₂,
DTPA-Pro-Gln-gmCpa-Tyr-Gly-Asn-Gln-Trp-Ala-Val-Gly-His-Leu-Met-NH_{2,}
DTPA-Pro-Gln-geCpa-Tyr-Gly-Asn-Gln-Trp-Ala-Val-Gly-His-Leu-Met-NH_{2,}
DTPA-Pro-Gln-gpCpa-Tyr-Gly-Asn-Gln-Trp-Ala-Val-Gly-His-Leu-Met-NH_{2,}
DTPA-Pro-Gln-gCha-Tyr-Gly-Asn-Gln-Trp-Ala-Val-Gly-His-Leu-Met-NH₂,
DTPA-Pro-Gln-gmCha-Tyr-Gly-Asn-Gln-Trp-Ala-Val-Gly-His-Leu-Met-NH_{2,}
DTPA-Pro-Gln-geCha-Tyr-Gly-Asn-Gln-Trp-Ala-Val-Gly-His-Leu-Met-NH₂,
DTPA-Pro-Gln-gpCha-Tyr-Gly-Asn-Gln-Trp-Ala-Val-Gly-His-Leu-Met-NH₂,
DTPA-Pro-Gln-gChpa-Tyr-Gly-Asn-Gln-Trp-Ala-Val-Gly-His-Leu-Met-NH₂,
DTPA-Pro-Gln-gmChpa-Tyr-Gly-Asn-Gln-Trp-Ala-Val-Gly-His-Leu-Met-NH_{2,}
DTPA-Pro-Gln-geChpa-Tyr-Gly-Asn-Gln-Trp-Ala-Val-Gly-His-Leu-Met-NH_{2,}
DTPA-Pro-Gin-gpChpa-Tyr-Gly-Asn-Gln-Trp-Ala-Val-Gly-His-Leu-Met-NH₂,
DTPA-Pip-Gln-gCpa-Tyr-Gly-Asn-Gln-Trp-Ala-Val-Gly-His-Leu-Met-NH_{2,}
DTPA-Pip-Gln-gmCpa-Tyr-Gly-Asn-Gin-Trp-Ala-Val-Gly-His-Leu-Met-NH₂,
DTPA-Pip-Gln-geCpa-Tyr-Gly-Asn-Gln-Trp-Ala-Val-Gly-His-Leu-Met-NH_{2,}
DTPA-Pip-Gin-gpCpa-Tyr-Gly-Asn-Gln-Trp-Ala-Val-Gly-His-Leu-Met-NH₂,
DTPA-Pip-Gln-gCha-Tyr-Gly-Asn-Gln-Trp-Ala-Val-Gly-His-Leu-Met-NH₂,
DTPA-Pip-Gin-gmCha-Tyr-Gly-Asn-Gln-Trp-Ala-Val-Gly-His-Leu-Met-NH_{2.}
DTPA-Pip-Gln-geCha-Tyr-Gly-Asn-Gln-Trp-Ala-Val-Gly-His-Leu-Met-NH_{2,}
DTPA-Pip-Gln-gpCha-Tyr-Gly-Asn-Gln-Trp-Ala-Val-Gly-His-Leu-Met-NH₂,
DTPA-Pip-Gln-gChPa-Tyr-Gly-Asn-Gln-Trp-Ala-Val-Gly-His-Leu-Met-NH_{2,}
DTPA-Pip-Gln-gmChpa-Tyr-Gly-Asn-Gln-Trp-Ala-Val-Gly-His-Leu-Met-NH_{2,}
DTPA-Pip-Gln-geChpa-Tyr-Gly-Asn-Gln-Trp-Ala-Val-Gly-His-Leu-Met-NH₂,
DTPA-Pip-Gin-gpChpa-Tyr-Gly-Asn-Gin-Trp-Ala-Val-Gly-His-Leu-Met-NH₂,
DTPA-hPip-Gln-gCpa-Tyr-Gly-Asn-Gln-Trp-Ala-Val-Gly-His-Leu-Met-NH_{2,}
DTPA-hPip-Gln-gmCpa-Tyr-Gly-Asn-Gln-Trp-Ala-Val-Gly-His-Leu-Met-NH_{2,}
DTPA-hPip-Gln-geCpa-Tyr-Gly-Asn-Gln-Trp-Ala-Val-Gly-His-Leu-Met-NH_{2,}
DTPA-hPip-Gln-gpCpa-Tyr-Gly-Asn-Gln-Trp-Ala-Val-Gly-His-Leu-Met-NH₂,
DTPA-hPip-Gln-gCha-Tyr-Gly-Asn-Gln-Trp-Ala-Val-Gly-His-Leu-Met-NH₂,
DTPA-hPip-Gln-gmCha-Tyr-Gly-Asn-Gln-Trp-Ala-Val-Gly-His-Leu-Met-NH₂,
DTPA-hPip-Gln-geCha-Tyr-Gly-Asn-Gln-Trp-Ala-Val-Gly-His-Leu-Met-NH_{2,}
DTPA-hPip-Gln-gpCha-Tyr-Gly-Asn-Gln-Trp-Ala-Val-Gly-His-Leu-Met-NH_{2,}
DTPA-hPip-Gin-gChpa-Tyr-Gly-Asn-Gln-Trp-Ala-Val-Gly-His-Leu-Met-NH₂,
DTPA-hPip-Gln-gmChpa-Tyr-Gly-Asn-Gln-Trp-Ala-Val-Gly-His-Leu-Met-NH₂,
DTPA-hPip-Gln-geChpa-Tyr-Gly-Asn-Gln-Trp-Ala-Val-Gly-His-Leu-Met-NH₂,
DTPA-hPip-Gln-gpChpa-Tyr-Gly-Asn-Gln-Trp-Ala-Val-Gly-His-Leu-Met-NH₂,
DTPA-Moc-Gln-gCpa-Tyr-Gly-Asn-Gln-Trp-Ala-Val-Gly-His-Leu-Met-NH_{2,}
DTPA-Moc-Gln-gmCpa-Tyr-Gly-Asn-Gln-Trp-Ala-Val-Gly-His-Leu-Met-NH_{2,}
DTPA-Moc-Gln-geCpa-Tyr-Gly-Asn-Gln-Trp-Ala-Val-Gly-His-Leu-Met-NH₂,
DTPA-Moc-Gln-gpCpa-Tyr-Gly-Asn-Gln-Trp-Ala-Val-Gly-His-Leu-Met-NH₂,
DTPA-Moc-Gln-gCha-Tyr-GIy-Asn-Gln-Trp-Ala-Val-Gly-His-Leu-Met-NH₂,
DTPA-Moc-Gln-gmCha-Tyr-Gly-Asn-Gln-Tcp-Ala-Val-Gly-His-Leu-Met-NH₂,
DTPA-Moc-Gln-geCha-Tyr-Gly-Asn-Gln-Trp-Ala-Val-Gly-His-Leu-Met-NH_{2,}
DTPA-Moc-Gln-gpCha-Tyr-Gly-Asn-Gln-Trp-Ala-Val-Gly-His-Leu-Met-NH_{2,}
DTPA-Moc-Gln-gChpa-Tyr-Gly-Asn-Gln-Trp-Ala-Val-Gly-His-Leu-Met-NH₂,
DTPA-Moc-Gln-gmChpa-Tyr-Gly-Asn-Gln-Trp-Ala-Val-Gly-His-Leu-Met-NH_{2,}
DTPA-Moc-Gln-geChpa-Tyr-Gly-Asn-Gln-Trp-Ala-Val-Gly-His-Leu-Met-NH_{2,}
DTPA-Moc-Gln-gpChpa-Tyr-Gly-Asn-Gln-Trp-Ala-Val-Gly-His-Leu-Met-NH₂,
DTPA-Mtc-Gln-gCpa-Tyr-Gly-Asn-Gln-Trp-Ala-Val-Gly-His-Leu-Met-NH_{2,}
DTPA-Mtc-Gln-gmCpa-Tyr-Gly-Asn-Gln-Trp-Ala-Val-Gly-His-Leu-Met-NH_{2,}
DTPA-Mtc-Gln-geCpa-Tyr-Gly-Asn-Gln-Trp-Ala-Val-Gly-His-Leu-Met-NH_{2,}
DTPA-Mtc-Gln-gpCpa-Tyr-Gly-Asn-Gln-Trp-Ala-Val-Gly-His-Leu-Met-NH_{2,}
DTPA-Mtc-Gln-gCha-Tyr-Gly-Asn-Gln-Trp-Ala-Val-Gly-His-Leu-Met-NH_{2,}
DTPA-Mtc-Gln-gmCha-Tyr-Gly-Asn-Gln-Trp-Ala-Val-Gly-His-Leu-Met-NH_{2,}
DTPA-Mtc-Gln-geCha-Tyr-Gly-Asn-Gln-Trp-Ala-Val-Gly-His-Leu-Met-NH_{2,}
DTPA-Mtc-Gln-gpCha-Tyr-Gly-Asn-Gln-Trp-Ala-Val-Gly-His-Leu-Met-NH₂,
DTPA-Mtc-Gln-gChpa-Tyr-Gly-Asn-Gln-Trp-Ala-Val-Gly-His-Leu-Met-NH₂,
DTPA-Moc-Gln-gmChpa-Tyr-Gly-Asn-Gln-Trp-Ala-Val-Gly-His-Leu-Met-NH₂,
DTPA-Moc-Gin-geChpa-Tyr-Gly-Asn-Gin-Trp-Ala-Val-Gly-His-Leu-Met-NH₂,
and
DTPA-Moc-Gln-gpChpa-Tyr-Gly-Asn-Gln-Trp-Ala-Val-Gly-His-Leu-Met-NH₂.

## Patentansprüche

1. Peptid AA₁-Gln-AA₃-Leu-Gly-Asn-Gln-Trp-Ala-Val-Gly-His-Leu-Met-NH₂, wobei AA₁ eine zyklische Aminosäure ist, mit der Formel 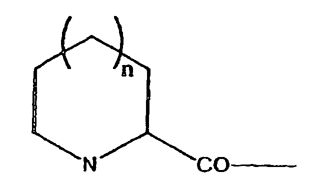 wobei n = 0 bis 5; und
wobei AA₃ ausgewählt ist aus der Gruppe bestehend aus Arg, 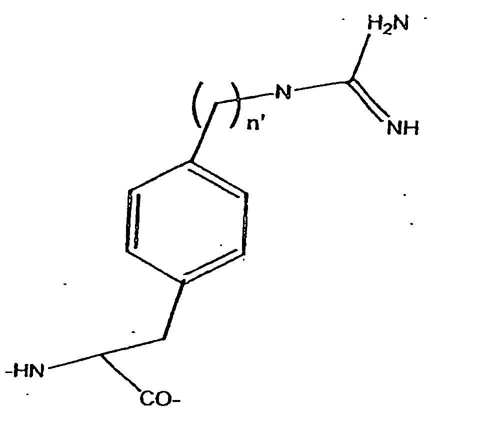 wobei n' = 0 bis 2, und 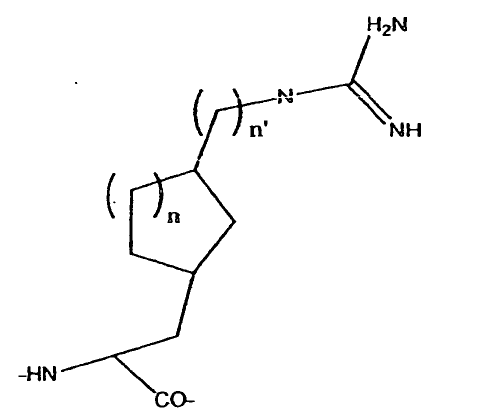 wobei n = 1 bis 3 und n' = 0 bis 3.

2. Peptid nach Anspruch 1, wobei AA₁ aus der Gruppe bestehend aus Pro, Pip, hPip, Moc und Mtc ausgewählt ist, und AA₃ aus der Gruppe bestehend aus Arg, gPhe, gmPhe, gePhe, gCpa, gmCpa, gpCpa, gCha, gmCha, geCha, gpCha, gChpa, gmChpa, geChpa und gpChpa ausgewählt ist.

3. Peptid nach Anspruch 1, wobei AA₁ aus der Gruppe bestehend aus L-His-AA₁, D-His-AA₁, L-His-Asp-AA₁, und D-His-Asp-AA₁ ausgewählt ist.

4. Peptid nach Anspruch 1, wobei der zyklische Ring weiter ein Heteroatom einschließt, das aus der Gruppe bestehend aus O, S und N-R ausgewählt ist, wobei R = Alkylgruppen C₁-C₆ normal oder verzweigt.

5. Peptid nach Anspruch 1, wobei die -CO- Gruppe ersetzt ist durch eine verzweigte oder normale Alkylkette mit oder ohne intervenierende Heteroatome und eine reaktive Gruppe ausgewählt aus der Gruppe bestehend aus COOH, NCS, NCO und OCOX, wobei X ein reaktiver Rest ist.

6. Peptid nach Anspruch 1, das weiter ein diagnostisches oder therapeutisches Radionuklid einschließt, das an das Peptid durch einen Chelatbildner gekoppelt ist.

7. Peptid nach Anspruch 6, wobei das diagnostische Radionuklid aus der Gruppe bestehend aus ^{133m}In ^{99m}Tc ⁶⁷Ga, ⁶⁸Ga, ⁷²As, ¹¹¹In, ⁹⁷Ru, ²⁰³Pb, ⁶²Cu, ⁶⁴Cu, ^{133m}In, ⁵¹Cr, ¹⁵⁷Gd, ¹²³I, ¹²⁴I, ¹³¹I, ⁷⁵Br, ⁷⁶Br, ⁷⁷Br und ⁸²Br ausgewählt ist.

8. Peptid nach Anspruch 6, wobei das therapeutische Radionuklid aus der Gruppe bestehend aus ¹⁸⁶Re, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁹⁹Au, ¹⁶¹Tb, ¹⁰⁹Pd, ¹⁸⁸Re, ⁷⁷As, ¹⁶⁶Dy, ¹⁶⁶Ho, ¹⁴⁹Pm, ¹⁵¹Pm, ¹⁵³Sm, ¹⁵⁹Gd, ¹⁷²Tm, ¹⁶⁹Yb, ¹⁷⁵Yb, ¹⁷⁷Lu, ¹⁰⁵Rh, ¹¹¹Ag, ¹³¹I, ¹²⁹I und ^{177m}Sn ausgewählt ist.

9. Peptid nach Anspruch 6, wobei der Chelatbildner aus der Gruppe bestehend aus DTPA, DTPA', DOTA und NₜS₄₋ₜ ausgewählt ist.

10. Pharmazeutische Zusammensetzung, die das markierte Peptid nach Anspruche 6 bis 9 und eine Trägersubstanz oder einen Zusatzstoff umfasst.

11. Kit zur Diagnose von Brust- oder Prostatatumoren oder Metastasen solcher Tumoren in einem Menschen, umfassend
(a) ein Peptid nach Anspruch 1;
(b) ein Radioisotop; und
(c) Zusatzstoffe geeignet für die Bindung des Radioisotops an das Peptid und Verabreichen der resultierenden Zusammensetzung an einen Menschen.

12. Peptid nach Anspruch 1 zur Verwendung in der Diagnose.

13. Verwendung eines Peptids nach Anspruch 1 zur Herstellung eines Medikaments zur Diagnose von GRP-rezeptorpositiven Tumoren.

14. Verwendung eines Peptids nach Anspruch 1 zur Herstellung eines Medikaments zur Diagnose von Prostatakrebs, Brustkrebs und Metastasen solcher Krebsarten.

15. Kit zur Behandlung von Brust- oder Prostatatumoren oder Metastasen solcher Tumoren in einem Menschen, umfassend
(a) ein Peptid nach Anspruch 1;
(b) ein Radioisotop; und
(c) Zusatzstoffe geeignet für die Bindung des Radioisotops an das Peptid und Verabreichen der resultierenden Zusammensetzung an einen Menschen.

16. Peptid nach Anspruch 1 zur Verwendung in der Therapie.

17. Verwendung eines Peptids nach Anspruch 1 zur Herstellung eines Medikaments zur Behandlung von GRP-rezeptorpositiven Tumoren.

18. Verwendung eines Peptids nach Anspruch 1 zur Herstellung eines Medikaments zur Behandlung von Prostatakrebs, Brustkrebs und Metastasen solcher Krebsarten.

19. Peptid ausgewählt aus der Gruppe bestehend aus:
DTPA-Pip-Gln-Arg-Tyr-Gly-Asn-Gln-Trp-Ala-Val-Gly-His-Leu-Met-NH_{2,}
DTPA-hPip-Gln-Arg-Tyr-Gly-Asn-Gln-Trp-Ala-Val-Gly-His-Leu-Met-NH_{2,}
DTPA-Pro-Gln-Arg-Tyr-Gly-Asn-Gln-Trp-Ala-Val-Gly-His-Leu-Nle-NH_{2,}
DTPA-Pip-Gln-Arg-Tyr-Gly-Asn-Gln-Trp-Ala-Val-Gly-His-Leu-Nle-NH_{2,}
DTPA-hPip-Gln-Arg-Tyr-Gly-Asn-Gln-Trp-Ala-Val-Gly-His-Leu-Nle-NH₂,
DTPA-Moc-Gln-Arg-Tyr-Gly-Asn-Gln-Trp-Ala-Val-Gly-His-Leu-Met-NH_{2,}
DTPA-Mtc-Gln-Arg-Tyr-Gly-Asn-Gln-Trp-Ala-Val-Gly-His-Leu-Met-NH_{2,}
DTPA-Pro-Gln-gPhe-Tyr-Gly-Asn-Gln-Trp-Ala-Val-Gly-His-Leu-Met-NH_{2,}
DTPA-Pro-Gln-gmPhe-Tyr-Gly-Asn-Gln-Trp-Ala-Val-Gly-His-Leu-Met-NH_{2,}
DTPA-Pro-Gln-gePhe-Tyr-Gly-Asn-Gln-Trp-Ala-Val-Gly-His-Leu-Met-NH_{2,}
DTPA-Pip-Gln-gPhe-Tyr-Gly-Asn-Gln-Trp-Ala-Val-Gly-His-Leu-Met-NH_{2,}
DTPA-Pip-Gln-gmPhe-Tyr-Gly-Asn-Gln-Trp-Ala-Val-Gly-His-Leu-Met-NH_{2,}
DTPA-Pip-Gln-gePhe-Tyr-Gly-Asn-Gln-Trp-Ala-Val-Gly-His-Leu-Met-NH_{2,}
DTPA-hPip-Gin-gPhe-Tyr-Gly-Asn-Gln-Trp-Ala-Val-Gly-His-Leu-Met-NH_{2,}
DTPA-Moc-Gln-gmPhe-Tyr-Gly-Asn-Gln-Trp-Ala-Val-Gly-His-Leu-Met-NH_{2,}
DTPA-Mtc-Gln-gePhe-Tyr-Gly-Asn-Gln-Trp-Ala-Val-Gly-His-Leu-Met-NH_{2,}
DTPA-Pro-Gln-gCpa-Tyr-Gly-Asn-Gln-Trp-Ala-Val-Gly-His-Leu-Met-NH_{2,}
DTPA-Pro-Gln-gmCpa-Tyr-Gly-Asn-Gln-Trp-Ala-Val-Gly-His-Leu-Met-NH_{2,}
DTPA-Pro-Gln-geCpa-Tyr-Gly-Asn-Gln-Trp-Ala-Val-Gly-His-Leu-Met-NH_{2,}
DTPA-Pro-Gln-gpCpa-Tyr-Gly-Asn-Gln-Trp-Ala-Val-Gly-His-Leu-Met-NH_{2,}
DTPA-Pro-Gln-gCha-Tyr-Gly-Asn-Gln-Trp-Ala-Val-Gly-His-Leu-Met-NH_{2,}
DTPA-Pro-Gln-gmCha-Tyr-Gly-Asn-Gln-Trp-Ala-Val-Gly-His-Leu-Met-NH_{2,}
DTPA-Pro-Gln-geCha-Tyr-Gly-Asn-Gln-Trp-Ala-Val-Gly-His-Leu-Met-NH_{2,}
DTPA-Pro-Gln-gpCha-Tyr-Gly-Asn-Gln-Trp-Ala-Val-Gly-His-Leu-Met-NH_{2,}
DTPA-Pro-Gln-gChpa-Tyr-Gly-Asn-Gln-Trp-Ala-Val-Gly-His-Leu-Met-NH_{2,}
DTPA-Pro-Gln-gmChpa-Tyr-Gly-Asn-Gln-Trp-Ala-Val-Gly-His-Leu-Met-NH₂,
DTPA-Pro-Gln-geChpa-Tyr-Gly-Asn-Gln-Trp-Ala-Val-Gly-His-Leu-Met-NH_{2,}
DTPA-Pro-Gln-gpChpa-Tyr-Gly-Asn-Gln-Trp-Ala-Val-Gly-His-Leu-Met-NH_{2,}
DTPA-Pip-Gln-gCpa-Tyr-Gly-Asn-Gln-Trp-Ala-Val-Gly-His-Leu-Met-NH_{2,}
DTPA-Pip-Gln-gmCpa-Tyr-Gly-Asn-Gln-Trp-Ala-Val-Gly-His-Leu-Met-NH_{2,}
DTPA-Pip-Gln-geCpa-Tyr-Gly-Asn-Gln-Trp-Ala-Val-Gly-His-Leu-Met-NH₂,
DTPA-Pip-Gln-gpCpa-Tyr-Gly-Asn-Gln-Trp-Ala-Val-Gly-His-Leu-Met-NH_{2,}
DTPA-Pip-Gln-gCha-Tyr-Gly-Asn-Gln-Trp-Ala-Val-Gly-His-Leu-Met-NH_{2,}
DTPA-Pip-Gln-gmCha-Tyr-Gly-Asn-Gln-Trp-Ala-Val-Gly-His-Leu-Met-NH_{2,}
DTPA-Pip-Gln-geCha-Tyr-Gly-Asn-Gln-Trp-Ala-Val-Gly-His-Leu-Met-NH_{2,}
DTPA-Pip-Gln-gpCha-Tyr-Gly-Asn-Gln-Trp-Ala-Val-Gly-His-Leu-Met-NH_{2,}
DTPA-Pip-Gln-gChPa-Tyr-Gly-Asn-Gln-Trp-Ala-Val-Gly-His-Leu-Met-NH_{2,}
DTPA-Pip-Gln-gmChpa-Tyr-Gly-Asn-Gln-Trp-Ala-Val-Gly-His-Leu-Met-NH_{2,}
DTPA-Pip-Gln-geChpa-Tyr-Gly-Asn-Gln-Trp-Ala-Val-Gly-His-Leu-Met-NH_{2,}
DTPA-Pip-Gln-gpChpa-Tyr-Gly-Asn-Gln-Trp-Ala-Val-Gly-His-Leu-Met-NH_{2,}
DTPA-hPip-Gln-gCpa-Tyr-Gly-Asn-Gln-Trp-Ala-Val-Gly-His-Leu-Met-NH_{2,}
DTPA-hPip-Gln-gmCpa-Tyr-Gly-Asn-Gln-Trp-Ala-Val-Gly-His-Leu-Met-NH_{2,}
DTPA-hPip-Gln-geCpa-Tyr-Gly-Asn-Gln-Trp-Ala-Val-Gly-His-Leu-Met-NH_{2,}
DTPA-hPip-Gln-gpCpa-Tyr-Gly-Asn-Gln-Trp-Ala-Val-Gly-His-Leu-Met-NH_{2,}
DTPA-hPip-Gln-gCha-Tyr-Gly-Asn-Gln-Trp-Ala-Val-Gly-His-Leu-Met-NH_{2,}
DTPA-hPip-Gln-gmCha-Tyr-Gly-Asn-Gln-Trp-Ala-Val-Gly-His-Leu-Met-NH_{2,}
DTPA-hPip-Gln-geCha-Tyr-Gly-Asn-Gln-Trp-Ala-Val-Gly-His-Leu-Met-NH_{2,}
DTPA-hPip-Gln-gpCha-Tyr-Gly-Asn-Gln-Trp-Ala-Val-Gly-His-Leu-Met-NH_{2,}
DTPA-hPip-Gln-gChpa-Tyr-Gly-Asn-Gln-Trp-Ala-Val-Gly-His-Leu-Met-NH_{2,}
DTPA-hPip-Gln-gmChpa-Tyr-Gly-Asn-Gln-Trp-Ala-Val-Gly-His-Leu-Met-NH₂,
DTPA-hPip-Gln-geChpa-Tyr-Gly-Asn-Gln-Trp-Ala-Val-Gly-His-Leu-Met-NH_{2,}
DTPA-hPip-Gln-gpChpa-Tyr-Gly-Asn-Gln-Trp-Ala-Val-Gly-His-Leu-Met-NH_{2,}
DTPA-Moc-Gln-gCpa-Tyr-Gly-Asn-Gln-Trp-Ala-Val-Gly-His-Leu-Met-NH_{2,}
DTPA-Moc-Gln-gmCpa-Tyr-Gly-Asn-Gln-Trp-Ala-Val-Gly-His-Leu-Met-NH_{2,}
DTPA-Moc-Gln-geCpa-Tyr-Gly-Asn-Gln-Trp-Ala-Val-Gly-His-Leu-Met-NH_{2,}
DTPA-Moc-Gln-gpCpa-Tyr-Gly-Asn-Gln-Trp-Ala-Val-Gly-His-Leu-Met-NH_{2,}
DTPA-Moc-Gln-gCha-Tyr-Gly-Asn-Gln-Trp-Ala-Val-Gly-His-Leu-Met-NH_{2,}
DTPA-Moc-Gln-gmCha-Tyr-Gly-Asn-Gln-Trp-Ala-Val-Gly-His-Leu-Met-NH_{2,}
DTPA-Moc-Gln-geCha-Tyr-Gly-Asn-Gln-Trp-Ala-Val-Gly-His-Leu-Met-NH_{2,}
DTPA-Moc-Gln-gpCha-Tyr-Gly-Asn-Gln-Trp-Ala-Val-Gly-His-Leu-Met-NH_{2,}
DTPA-Moc-Gln-gChpa-Tyr-Gly-Asn-Gln-Trp-Ala-Val-Gly-His-Leu-Met-NH_{2,}
DTPA-Moc-Gln-gmChpa-Tyr-Gly-Asn-Gln-Trp-Ala-Val-Gly-His-Leu-Met-NH_{2,}
DTPA-Moc-Gln-geChpa-Tyr-Gly-Asn-Gln-Trp-Ala-Val-Gly-His-Leu-Met-NH_{2,}
DTPA-Moc-Gln-gpChpa-Tyr-Gly-Asn-Gln-Trp-Ala-Val-Gly-His-Leu-Met-NH_{2,}
DTPA-Mtc-Gln-gCpa-Tyr-Gly-Asn-Gln-Trp-Ala-Val-Gly-His-Leu-Met-NH_{2,}
DTPA-Mtc-Gln-gmCpa-Tyr-Gly-Asn-Gln-Trp-Ala-Val-Gly-His-Leu-Met-NH_{2,}
DTPA-Mtc-Gln-geCpa-Tyr-Gly-Asn-Gln-Trp-Ala-Val-Gly-His-Leu-Met-NH_{2,}
DTPA-Mtc-Gln-gpCpa-Tyr-Gly-Asn-Gln-Trp-Ala-Val-Gly-His-Leu-Met-NH_{2,}
DTPA-Mtc-Gln-gCha-Tyr-Gly-Asn-Gln-Trp-Ala-Val-Gly-His-Leu-Met-NH_{2,}
DTPA-Mtc-Gln-gmCha-Tyr-Gly-Asn-Gln-Trp-Ala-Val-Gly-His-Leu-Met-NH_{2,}
DTPA-Mtc-Gln-geCha-Tyr-Gly-Asn-Gln-Trp-Ala-Val-Gly-His-Leu-Met-NH_{2,}
DTPA-Mtc-Gln-gpCha-Tyr-Gly-Asn-Gln-Trp-Ala-Val-Gly-His-Leu-Met-NH_{2,}
DTPA-Mtc-Gln-gChpa-Tyr-Gly-Asn-Gln-Trp-Ala-Val-Gly-His-Leu-Met-NH_{2,}
DTPA-Moc-Gln-gmChpa-Tyr-Gly-Asn-Gln-Trp-Ala-Val-Gly-His-Leu-Met-NH_{2,}
DTPA-Moc-Gln-geChpa-Tyr-Gly-Asn-Gln-Trp-Ala-Val-Gly-His-Leu-Met-NH_{2,}
und
DTPA-Moc-Gln-gpChpa-Tyr-Gly-Asn-Gln-Trp-Ala-Val-Gly-His-Leu-Met-NH₂.

## Revendications

1. Peptide AA₁-Gln-AA₃-Leu-Gly-Asn-Gln-Trp-Ala-Val-Gly-His-Leu-Met-NH₂,
dans lequel AA₁ représente un aminoacide cyclique de formule 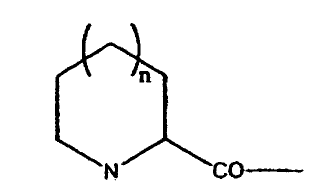 dans laquelle n a une valeur de 0 à 5 ; et
dans laquelle AA₃ est choisi dans le groupe consistant en Arg, 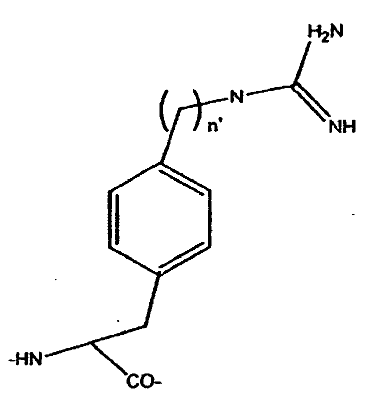 formule dans laquelle n' a une valeur de 0 à 2, et 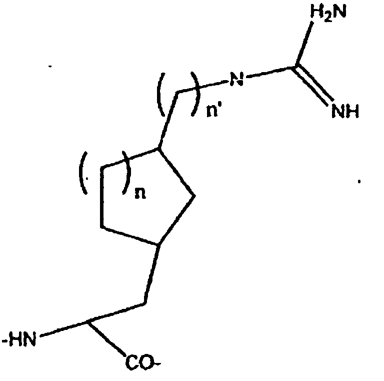 formule dans laquelle n à une valeur de 1 à 3 et n' a une valeur de 0 à 3.

2. Peptide suivant la revendication 1, dans lequel AA1 est choisi dans le groupe consistant en Pro, Pip, hPip, Moc et Mtc, et AA₃ est choisi dans le groupe consistant en Arg, gPhe, gmPhe, gePhe, gCpa, gmCpa, gpCpa, gCha, gmCha, geCha, gpCha, gChpa, gmChpa, geChpa et gpChpa.

3. Peptide suivant la revendication 1, dans lequel AA₁ est choisi dans le groupe consistant en L-His-AA₁, D-His-AA₁, L-His-Asp-AA₁ et D-His-Asp-AA₁.

4. Peptide suivant la revendication 1, dans lequel le noyau cyclique comprend en outre un hétéroatome choisi dans le groupe consistant en O, S et N-R, dans lequel R représente des groupes alkyle en C₁ à C₆, normaux ou ramifiés.

5. Peptide suivant la revendication 1, dans lequel le groupe -CO- est remplacé par une chaîne alkyle ramifiée ou normale avec ou sans hétéroatomes intermédiaires et un groupe réactif choisi dans le groupe consistant en des groupes COOH, NCS, NCO et OCOX, dans lequel X représente un groupement réactif.

6. Peptide suivant la revendication 1, comprenant en outre un radionucléide de diagnostic ou thérapeutique couplé au peptide par un agent chélatant.

7. Peptide suivant la revendication 6, dans lequel le radionucléide de diagnostic est choisi dans le groupe consistant en ^{133m}In, ^{99m}Tc, ⁶⁷Ga, ⁶⁸Ga, ⁷²As, ¹¹¹In, ⁹⁷Ru, ²⁰³Pb, ⁶²Cu, ⁶⁴Cu, ^{133m}In, ⁵¹Cr, ¹⁵⁷Gd, ¹²³I, ¹²⁴I, ¹³¹I, ⁷⁵Br, ⁷⁶Br, ⁷⁷Br et ⁸²Br.

8. Peptide suivant la revendication 6, dans lequel le radionucléide thérapeutique est choisi dans le groupe consistant en ¹⁸⁶Re, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁹⁹Au, ¹⁶¹Tb, ¹⁰⁹Pd, ¹⁸⁸Re, ⁷⁷As, ¹⁶⁶Dy, ¹⁶⁶Ho, ¹⁴⁹Pm, ¹⁵¹Pm, ¹⁵³Sm, ¹⁵⁹Gd, ¹⁷²Tm, ¹⁶⁹Yb, ¹⁷⁵Yb, ¹⁷⁷Lu, ¹⁰⁵Rh, ¹¹¹Ag, ¹³¹I, ¹²⁹I et ^{177m}Sn.

9. Peptide suivant la revendication 6, dans lequel l'agent chélatant est choisi dans le groupe consistant en DTPA, DTPA', DOTA et NₜS₄₋ₜ.

10. Composition pharmaceutique comprenant le peptide marqué des revendications 6 à 9 et une matière de support ou un adjuvant.

11. Kit pour le diagnostic de tumeurs du sein ou de la prostate ou de métastases de ces tumeurs chez un être humain, comprenant
(a) un peptide suivant la revendication 1 ;
(b) un radioisotope ; et
(c) des adjuvants convenables pour lier le radioisotope au peptide et administrer l'association résultante à un être humain.

12. Peptide suivant la revendication 1, destiné à être utilisé en diagnostic.

13. Utilisation d'un peptide suivant la revendication 1 dans la production d'un médicament destiné au diagnostic de tumeurs positives pour le récepteur de GRP.

14. Utilisation d'un peptide suivant la revendication 1 dans la production d'un médicament destiné au diagnostic du cancer de la prostate, du cancer du sein et de métastases de ces cancers.

15. Kit pour le traitement de tumeurs du sein ou de la prostate ou de métastases de ces tumeurs chez un être humain, comprenant
(a) un peptide suivant la revendication 1 ;
(b) un radioisotope ; et
(c) des adjuvants convenables pour lier le radioisotope au peptide et administrer l'association résultante à un être humain.

16. Peptide suivant la revendication 1, destiné à être utilisé en thérapeutique.

17. Utilisation d'un peptide suivant la revendication 1 dans la production d'un médicament destiné au traitement de tumeurs positives pour le récepteur de GRP.

18. Utilisation d'un peptide suivant la revendication 1 dans la production d'un médicament destiné au traitement du cancer de la prostate, du cancer du sein et de métastases de ces cancers.

19. Peptide choisi dans le groupe consistant en
DTPA-Pip-Gln-Arg-Tyr-Gly-Asn-Gln-Trp-Ala-Val-Gly-His-Leu-Met-NH_{2,}
DTPA-hPip-Gln-Arg-Tyr-Gly-Asn-Gln-Trp-Ala-Val-Gly-His-Leu-Met-NH_{2,}
DTPA-Pro-Gln-Arg-Tyr-Gly-Asn-Gln-Trp-Ala-Val-Gly-His-Leu-Nle-NH_{2,}
DTPA-Pip-Gln-Arg-Tyr-Gly-Asn-Gln-Trp-Ala-Val-Gly-His-Leu-Nle-NH_{2,}
DTPA-hPip-Gln-Arg-Tyr-Gly-Asn-Gln-Trp-Ala-Val-Gly-His-Leu-Nle-NH_{2,}
DTPA-Moc-Gln-Arg-Tyr-Gly-Asn-Gln-Trp-Ala-Val-Gly-His-Leu-Met-NH_{2,}
DTPA-Mtc-Gln-Arg-Tyr-Gly-Asn-Gln-Trp-Ala-Val-Gly-His-Leu-Met-NH_{2,}
DTPA-Pro-Gln-gPhe-Tyr-Gly-Asn-Gln-Trp-Ala-Val-Gly-His-Leu-Met-NH_{2,}
DTPA-Pro-Gln-gmPhe-Tyr-Gly-Asn-Gln-Trp-Ala-Val-Gly-His-Leu-Met-NH_{2,}
DTPA-Pre-Gln-gePhe-Tyr-Gly-Asn-Gln-Trp-Ala-Val-Gly-His-Leu-Met-NH_{2,}
DTPA-Pip-Gln-gPhe-Tyr-Gly-Asn-Gln-Trp-Ala-Val-Gly-His-Leu-Met-NH_{2,}
DTPA-Pip-Gln-gmPhe-Tyr-Gly-Asn-Gln-Trp-Ala-Val-Gly-His-Leu-Met-NH_{2,}
DTPA-Pip-Gln-gePhe-Tyr-Gly-Asn-Gln-Trp-Ala-Val-Gly-His-Leu-Met-NH_{2,}
DTPA-hPip-Gln-gPhe-Tyr-Gly-Asn-Gln-Trp-Ala-Val-Gly-His-Leu-Met-NH_{2,}
DTPA-Moc-Gln-gmPhe-Tyr-Gly-Asn-Gln-Trp-Ala-Val-Gly-His-Leu-Met-NH_{2,}
DTPA-Mtc-Gln-gePhe-Tyr-Gly-Asn-Gln-Trp-Ala-Val-Gly-His-Leu-Met-NH_{2,}
DTPA-Pro-Gln-gCpa-Tyr-Gly-Asn-Gln-Trp-Ala-Val-Gly-His-Leu-Met-NH_{2,}
DTPA-Pro-Gln-gmCpa-Tyr-Gly-Asn-Gln-Trp-Ala-Val-Gly-His-Leu-Met-NH_{2,}
DTPA-Pro-Gln-geCpa-Tyr-Gly-Asn-Gln-Trp-Ala-Val-Gly-His-Leu-Met-NH_{2,}
DTPA-Pro-Gln-gpCpa-Tyr-Gly-Asn-Gln-Trp-Ala-Val-Gly-His-Leu-Met-NH_{2,}
DTPA-Pro-Gln-gCha-Tyr-Gly-Asn-Gln-Trp-Ala-Val-Gly-His-Leu-Met-NH₂,
DTPA-Pro-Gln-gmCha-Tyr-Gly-Asn-Gln-Trp-Ala-Val-Gly-His-Leu-Met-NH₂,
DTPA-Pro-Gln-geCha-Tyr-Gly-Asn-Gln-Trp-Ala-Val-Gly-His-Leu-Met-NH₂,
DTPA-Pro-Gln-gpCha-Tyr-Gly-Asn-Gln-Trp-Ala-Val-Gly-His-Leu-Met-NH₂,
DTPA-Pro-Gln-gChpa-Tyr-Gly-Asn-Gln-Trp-Ala-Val-Gly-His-Leu-Met-NH₂,
DTPA-Pro-Gln-gmChpa-Tyr-Gly-Asn-Gln-Trp-Ala-Val-Gly-His-Leu-Met-NH₂,
DTPA-Pro-Gln-geChpa-Tyr-Gly-Asn-Gln-Trp-Ala-Val-Gly-His-Leu-Met-NH₂,
DTPA-Pro-Gln-gpChpa-Tyr-Gly-Asn-Gln-Trp-Ala-Val-Gly-His-Leu-Met-NH₂,
DTPA-Pip-Gln-gCpa-Tyr-Gly-Asn-Gln-Trp-Ala-Val-Gly-His-Leu-Met-NH₂,
DTPA-Pip-Gln-gmCpa-Tyr-Gly-Asn-Gln-Trp-Ala-Val-Gly-His-Leu-Met-NH₂,
DTPA-Pip-Gln-geCpa-Tyr-Gly-Asn-Gln-Trp-Ala-Val-Gly-His-Leu-Met-NH₂,
DTPA-Pip-Gln-gpCpa-Tyr-Gly-Asn-Gln-Trp-Ala-Val-Gly-His-Leu-Met-NH₂,
DTPA-Pip-Gln-gCha-Tyr-Gly-Asn-Gln-Trp-Ala-Val-Gly-His-Leu-Met-NH₂,
DTPA-Pip-Gln-gmCha-Tyr-Gly-Asn-Gln-Trp-Ala-Val-Gly-His-Leu-Met-NH₂,
DTPA-Pip-Gln-geCha-Tyr-Gly-Asn-Gln-Trp-Ala-Val-Gly-His-Leu-Met-NH₂,
DTPA-Pip-Gln-gpCha-Tyr-Gly-Asn-Gln-Trp-Ala-Val-Gly-His-Leu-Met-NH₂,
DTPA-Pip-Gln-gChPa-Tyr-Gly-Asn-Gln-Trp-Ala-Val-Gly-His-Leu-Met-NH₂,
DTPA-Pip-Gln-gmChpa-Tyr-Gly-Asn-Gln-Trp-Ala-Val-Gly-His-Leu-Met-NH₂,
DTPA-Pip-Gln-geChpa-Tyr-Gly-Asn-Gln-Trp-Ala-Val-Gly-His-Leu-Met-NH₂,
DTPA-Pip-Gln-gpChpa-Tyr-Gly-Asn-Gln-Trp-Ala-Val-Gly-His-Leu-Met-NH₂,
DTPA-hPip-Gln-gCpa-Tyr-Gly-Asn-Gln-Trp-Ala-Val-Gly-His-Leu-Met-NH₂,
DTPA-hPip-Gln-gmCpa-Tyr-Gly-Asn-Gln-Trp-Ala-Val-Gly-His-Leu-Met-NH₂,
DTPA-hPip-Gln-geCpa-Tyr+Gly-Asn-Gln-Trp-Ala-Val-Gly-His-Leu-Met-NH₂,
DTPA-hPip-Gln-gpCpa-Tyr-Gly-Asn-Gln-Trp-Ala-Val-Gly-His-Leu-Met-NH₂,
DTPA-hPip-Gln-gCha-Tyr-Gly-Asn-Gln-Trp-Ala-Val-Gly-His-Leu-Met-NH₂,
DTPA-hPip-Gln-gmCha-Tyr-Gly-Asn-Gln-Trp-Ala-Val-Gly-His-Leu-Met-NH₂,
DTPA-hPip-Gln-geCha-Tyr-Gly-Asn-Gln-Trp-Ala-Val-Gly-His-Leu-Met-NH₂,
DTPA-hPip-Gln-gpCha-Tyr-Gly-Asn-Gln-Trp-Ala-Val-Gly-His-Leu-Met-NH₂,
DTPA-hPip-Gln-gChpa-Tyr-Gly-Asn-Gln-Trp-Ala-Val-Gly-His-Leu-Met-NH₂,
DTPA-hPip-Gln-gmChpa-Tyr-Gly-Asn-Gln-Trp-Ala-Val-Gly-His-Leu-Met-NH₂,
DTPA-hPip-Gln-geChpa-Tyr-Gly-Asn-Gln-Trp-Ala-Val-Gly-His-Leu-Met-NH_{2,}
DTPA-hPip-Gln-gpChpa-Tyr-Gly-Asn-Gln-Trp-Ala-Val-Gly-His-Leu-Met-NH_{2,}
DTPA-Moc-Gln-gCpa-Tyr-Gly-Asn-Gln-Trp-Ala-Val-Gly-His-Leu-Met-NH_{2,}
DTPA-Moc-Gln-gmCpa-Tyr-Gly-Asn-Gln-Trp-Ala-Val-Gly-His-Leu-Met-NH_{2,}
DTPA-Moc-Gln-geCpa-Tyr-Gly-Asn-Gln-Trp-Ala-Val-Gly-His-Leu-Met-NH_{2,}
DTPA-Moc-Gln-gpCpa-Tyr-Gly-Asn-Gln-Trp-Ala-Val-Gly-His-Leu-Met-NH_{2,}
DTPA-Moc-Gln-gCha-Tyr-Gly-Asn-Gln-Trp-Ala-Val-Gly-His-Leu-Met-NH_{2,}
DTPA-Moc-Gln-gmCha-Tyr-Gly-Asn-Gln-Trp-Ala-Val-Gly-His-Leu-Met-NH_{2,}
DTPA-Moc-Gln-geCha-Tyr-Gly-Asn-Gln-Trp-Ala-Val-Gly-His-Leu-Met-NH_{2,}
DTPA-Moc-Gln-gpCha-Tyr-Gly-Asn-Gln-Trp-Ala-Val-Gly-His-Leu-Met-NH_{2,}
DTPA-Moc-Gln-gChpa-Tyr-Gly-Asn-Gln-Trp-Ala-Val-Gly-His-Leu-Met-NH_{2,}
DTPA-Moc-Gln-gmChpa-Tyr-Gly-Asn-Gln-Trp-Ala-Val-Gly-His-Leu-Met-NH_{2,}
DTPA-Moc-Gln-geChpa-Tyr-Gly-Asn-Gln-Trp-Ala-Val-Gly-His-Leu-Met-NH_{2,}
DTPA-Moc-Gln-gpChpa-Tyr-Gly-Asn-Gln-Trp-Ala-Val-Gly-His-Leu-Met-NH_{2,}
DTPA-Mtc-Gln-gCpa-Tyr-Gly-Asn-Gln-Trp-Ala-Val-Gly-His-Leu-Met-NH_{2,}
DTPA-Mtc-Gln-gmCpa-Tyr-Gly-Asn-Gln-Trp-Ala-Val-Gly-His-Leu-Met-NH_{2,}
DTPA-Mtc-Gln-geCpa-Tyr-Gly-Asn-Gln-Trp-Ala-Val-Gly-His-Leu-Met-NH_{2,}
DTPA-Mtc-Gln-gpCpa-Tyr-Gly-Asn-Gln-Trp-Ala-Val-Gly-His-Leu-Met-NH_{2,}
DTPA-Mtc-Gln-gCha-Tyr-Gly-Asn-Gln-Trp-Ala-Val-Gly-His-Leu-Met-NH_{2,}
DTPA-Mtc-Gln-gmCha-Tyr-Gly-Asn-Gln-Trp-Ala-Val-Gly-His-Leu-Met-NH_{2,}
DTPA-Mtc-Gln-geCha-Tyr-Gly-Asn-Gln-Trp-Ala-Val-Gly-His-Leu-Met-NH_{2,}
DTPA-Mtc-Gln-gpCha-Tyr-Gly-Asn-Gln-Trp-Ala-Val-Gly-His-Leu-Met-NH_{2,}
DTPA-Mtc-Gln-gChpa-Tyr-Gly-Asn-Gln-Trp-Ala-Val-Gly-His-Leu-Met-NH_{2,}
DTPA-Moc-Gln-gmChpa-Tyr-Gly-Asn-Gln-Trp-Ala-Val-Gly-His-Leu-Met-NH_{2,}
DTPA-Moc-Gln-geChpa-Tyr-Gly-Asn-Gln-Trp-Ala-Val-Gly-His-Leu-Met-NH_{2,}
et
DTPA-Moc-Gln-gpChpa-Tyr-Gly-Asn-Gln-Trp-Aln-Val-Gly-His-Leu-Met-NH₂.
